(19) Europäisches Patentamt
European Patent Office
Office européen des brevets

(11) **EP 4 365 131 A1**

(12) **EUROPEAN PATENT APPLICATION**
published in accordance with Art. 153(4) EPC

(43) Date of publication:
**08.05.2024 Bulletin 2024/19**

(21) Application number: **22832502.3**

(22) Date of filing: **24.03.2022**

(51) International Patent Classification (IPC):
*C01B 13/11* (2006.01)    *A61L 9/015* (2006.01)
*G01N 27/27* (2006.01)    *G01N 27/416* (2006.01)
*B01D 46/10* (2006.01)

(52) Cooperative Patent Classification (CPC):
**A61L 9/015; B01D 46/10; C01B 13/11;
G01N 27/27; G01N 27/416**

(86) International application number:
**PCT/JP2022/013844**

(87) International publication number:
**WO 2023/276339 (05.01.2023 Gazette 2023/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.07.2021   JP 2021110571
20.07.2021   JP 2021119456
22.12.2021   JP 2021208080**

(71) Applicant: **Niterra Co., Ltd.
Nagoya-shi, Aichi 461-0005 (JP)**

(72) Inventors:
• **KITO, Shinichiro
Nagoya-shi, Aichi 461-0005 (JP)**
• **UEYAMA, Takeshi
Nagoya-shi, Aichi 461-0005 (JP)**
• **HATTORI, Yoichi
Nagoya-shi, Aichi 461-0005 (JP)**
• **NISHIYAMA, Hiroyuki
Nagoya-shi, Aichi 461-0005 (JP)**
• **HASUNUMA, Hideki
Nagoya-shi, Aichi 461-0005 (JP)**
• **YOKOYAMA, Takahiro
Nagoya-shi, Aichi 461-0005 (JP)**
• **OHTANI, Takayuki
Nagoya-shi, Aichi 461-0005 (JP)**

(74) Representative: **Grünecker Patent- und
Rechtsanwälte
PartG mbB
Leopoldstraße 4
80802 München (DE)**

(54) **OZONE GENERATOR**

(57)    An ozone generator (100) includes: a flow path (1) through which gas flows from an inlet (5) to an outlet (6); an ozone generation unit (3) disposed in the flow path (1); and an ozone sensor (4) disposed in the flow path (1) and upstream of the ozone generation unit (3). The flow path (1) has an upstream-side flow path (130) that forms a gas passing space (AR) located upstream of the ozone generation unit (3) and through which the gas flows from one side to another side in a predetermined direction. The inlet (5) is disposed closer to an outer circumferential portion (131) of the upstream-side flow path (130) than the ozone sensor (4).

Fig. 13

**Description**

TECHNICAL FIELD

The present invention relates to an ozone generator.

BACKGROUND ART

In the ozone generator disclosed in Patent Document 1, an ozone sensor is stored inside a casing (in a flow path). The ozone sensor is disposed in a linear flow path extending from an inlet toward an outlet.
Patent Document 2 discloses a device (small air purifier) for generating ozone. The device has a table as a base, and an air guide cover. An opening through which air in a room is taken in is formed between the table and the air guide cover, and a filter is arranged inside the opening.

PRIOR ART DOCUMENT

PATENT DOCUMENT

Patent Document 1: Japanese Patent Application Laid-Open (kokai) No. H05-49831
Patent Document 2: Japanese Patent Application Laid-Open (kokai) No. H07-323081

SUMMARY OF THE INVENTION

PROBLEM TO BE SOLVED BY THE INVENTION

In the ozone generator disclosed in Patent Document 1, the ozone sensor is disposed at a position where strong airflow is likely to occur. Therefore, the ozone sensor is exposed to the strong airflow, and measurement of an ozone concentration is likely to be influenced by the airflow.
The present invention provides an ozone generator capable of reducing influence of airflow in an ozone sensor.

MEANS FOR SOLVING THE PROBLEM

[1] An ozone generator according to one aspect of the present invention includes: a flow path through which gas flows from an inlet to an outlet; an ozone generation unit disposed in the flow path; and an ozone sensor disposed in the flow path and upstream of the ozone generation unit. The flow path has an upstream-side flow path that forms a gas passing space located upstream of the ozone generation unit and through which the gas flows from one side to another side in a predetermined direction. The inlet is disposed closer to an outer circumferential portion of the upstream-side flow path than the ozone sensor.
In such a configuration, since the inlet is disposed, in the upstream-side flow path in which gas flows from one side to the other side in a predetermined direction, closer to the outer circumferential portion than the ozone sensor, airflow from the inlet toward the other side in the predetermined direction is unlikely to go toward the ozone sensor located on the center side. Therefore, influence of airflow in the ozone sensor can be reduced.
[2] A filter is preferably disposed closer to a center of the upstream-side flow path than the inlet. The ozone sensor is preferably disposed closer to the center of the upstream-side flow path than the filter.
In such a configuration, gas passes through the filter, whereby the airflow is weakened. Therefore, influence of airflow in the ozone sensor can be further reduced.
[3] A fan for sending gas toward the outlet is preferably disposed downstream of the inlet and the ozone sensor. The ozone sensor preferably overlaps the fan when viewed in a rotation axis direction of the fan.
In such a configuration, since the direction from the inlet toward the fan is different from the rotation axis direction in which the ozone sensor and the fan overlap each other, airflow from the inlet toward the fan is unlikely to go toward the ozone sensor. Therefore, influence of airflow in the ozone sensor can be further reduced.
[4] The fan preferably has a rotor, and a blade portion protruding from the rotor in a radial direction. The ozone sensor is preferably disposed in a range extending from a center of the rotor over a distance of less than or equal to 1.2 times a diameter of the rotor, when viewed in the rotation axis direction.

In such a configuration, the ozone sensor can be easily disposed while avoiding a position at which flow of gas is unlikely to occur (position overlapping the rotor of the fan in the rotation axis direction). Therefore, influence of airflow in the ozone sensor can be further reduced.

[5] A fan for sending gas toward the outlet is preferably disposed downstream of the inlet and the ozone sensor. A sensing surface of the ozone sensor is preferably disposed closer to the inlet than a center of a length in the rotation axis direction of the fan between the inlet and the fan.

In such a configuration, the sensing surface of the ozone sensor can be easily kept away from airflow generated between the inlet and the fan. Therefore, the ozone sensor is much less likely to be influenced by airflow.

[6] A fan for sending gas toward the outlet is preferably disposed downstream of the inlet and the ozone sensor. The sensing surface of the ozone sensor is preferably disposed closer to the fan than the inlet in the rotation axis direction of the fan.

In such a configuration, the ozone sensor can be easily stored inside the ozone generator (on the downstream side), so that the size of the ozone generator can be easily reduced.

[7] A fan for sending gas toward the outlet is preferably disposed downstream of the inlet and the ozone sensor. The flow path preferably has a small-diameter flow path having a diameter less than a diameter of the outer circumferential portion. The fan is preferably disposed in the small-diameter flow path.

In such a configuration, the fan generates strong airflow in the small-diameter flow path that is relatively narrow, whereas the ozone sensor is disposed upstream of the fan. Therefore, the ozone sensor is less likely to be influenced by strong airflow.

[8] A plurality of the inlets is preferably arranged annularly along a circumferential direction of the outer circumferential portion.

In such a configuration, the ozone sensor can be less likely to be influenced by any flow of gas entering through the plurality of the inlets, while an intake amount of gas is sufficiently ensured.

[9] The ozone sensor is preferably an electrochemical gas sensor.

In such a configuration, measurement can be performed based on an electric current flowing according to an ozone gas concentration. Particularly, in an electrochemical gas sensor, when a sensing surface is exposed to airflow, the detection result is likely to be influenced by the airflow. Therefore, in a case where an electrochemical gas sensor is used, an effect of reducing influence of airflow in the ozone sensor is more effectively exhibited by applying the configuration of the ozone generator according to the present disclosure.

[10] The ozone sensor is preferably a gas sensor for detecting a concentration of ozone gas contained in a target gas, and preferably includes a first sensing element for electrochemically detecting a concentration of gas, a second sensing element for electrochemically detecting a concentration of gas, a first storage portion having, in its own structure, an internal space for storing the first sensing element, a first introduction inlet provided between the internal space and the flow path outside the first storage portion, and a moisture permeable film disposed at the first introduction inlet and configured to substantially prevent permeation of the ozone gas from the flow path to the internal space. The second sensing element is preferably disposed in the flow path into which water vapor and the ozone gas contained in the target gas flow.

In such a configuration, in the ozone sensor, the first sensing element detects an ozone gas concentration in a state where ozone gas is substantially removed from a target gas, so that influence of an electric current caused by difference in an amount of water vapor can be evaluated. Meanwhile, the second sensing element can detect a concentration of a target component containing water vapor and ozone gas. Thus, the ozone sensor can utilize a detection result of the first sensing element (result of evaluating of influence of an electric current caused by difference in an amount of water vapor) for detecting a concentration of the ozone gas by the second sensing element, which is advantageous in correctly detecting a concentration of the ozone gas.

[11] The moisture permeable film is preferably a water vapor permeable filter that allows permeation of water vapor from the flow path to the internal space, and substantially prevents permeation of the ozone gas from the flow path to the internal space.

In the ozone sensor, a function of allowing water vapor permeation of water vapor from the flow path to the internal space and substantially preventing permeation of the ozone gas from the flow path to the internal space can be achieved by the water vapor permeable filter.

[12] The ozone generator of the present invention includes an ozone generation unit for generating ozone, a flow path in which the ozone generation unit is disposed, and a filter disposed in the flow path. In this configuration, the flow path may have an outlet, an inlet disposed outward of an outer circumference of the outlet, a guiding flow path for guiding gas taken in through the inlet inwardly of an inner circumference of the inlet, and an outlet-side flow path for guiding the gas guided by the guiding flow path to the outlet. The filter may be disposed in the guiding flow path.

In this configuration, since the filter is disposed in the guiding flow path for guiding gas taken in through the inlet inwardly of the inner circumference of the inlet, the filter is invisible or cannot be easily seen from the inlet. Therefore, the design can be prevented from being degraded due to the filter being visible from the outside.

[13] The inlet may be disposed along an annular shape outward of an outer circumference of the outlet. The guiding flow path may be disposed along an annular shape, and may guide gas taken in through the inlet inwardly of an inner circumference of the inlet. The filter may be disposed along an annular shape in the guiding flow path.

In this configuration, gas is taken into the flow path through the entire circumference of the ozone generator, and a foreign object in the taken gas can be efficiently removed by the filter.

[14] The filter may be disposed along an intake direction of the inlet.

In this configuration, the filter is less likely to be visible from the inlet.

[15] The ozone generator may include: a filter frame to which the filter is mounted; a mounting portion to which the filter frame is mounted; an intake portion having the inlet; and an attachment portion to which the intake portion is detachably attached. The intake portion may prevent the filter frame from being removed from the mounting portion in a state where the intake portion is attached to the attachment portion, and allow the filter frame to be removed from the mounting portion in a state where the intake portion is detached from the attachment portion.

In this configuration, since the filter frame can be mounted and dismounted merely by detaching the intake portion, an operation of mounting and dismounting the filter frame is facilitated.

[16] The mounting portion may have a mounting groove into which the filter frame fits. The filter frame may be mounted to the mounting portion by fitting into the mounting groove.

In this configuration, the filter frame is easily mounted to and dismounted from the mounting portion.

[17] The ozone generator may include a filter frame to which a filter is mounted. The filter frame may have a frame body having an annular shape, and a tab protruding outwardly from an outer circumferential surface of the frame body.

In this configuration, even in a case where the inner circumferential surface of the frame body is adjacent to a member disposed inside and is not easily pinched, removal is facilitated by pinching the tab protruding from the outer circumferential surface of the frame body.

[18] The filter may be segmented into a plurality of segmented filters in the circumferential direction.

In this configuration, the filter is easily mounted.

[19] The ozone generator may include a filter frame having a mounting hole in which the filter is mounted. The inlet may be disposed along an annular shape outward of the outer circumference of the outlet. The guiding flow path may be disposed along an annular shape, and guide gas taken in through the inlet inwardly of the inner circumference of the inlet. The filter may be disposed along an annular shape in the guiding flow path. An opening width of the mounting hole in an axial direction of the inlet disposed along an annular shape may be greater than an opening width of the inlet in a direction orthogonal to the axial direction.

In this configuration, the collection area of the filter can be made greater than the opening area of the inlet. Therefore, a function of the filter for removing foreign objects can be prevented from being degraded while suppressing pressure loss due to the filter.

ADVANTAGEOUS EFFECTS OF THE INVENTION

The present invention can reduce influence of airflow in the ozone sensor.

BRIEF DESCRIPTION OF THE DRAWINGS

[Fig. 1] Fig. 1 is a perspective view of an ozone generator.
[Fig. 2] Fig. 2 is a perspective view of a cross-section of the ozone generator.
[Fig. 3] Fig. 3 is a cross-sectional view of the ozone generator on a cross-section different from the cross-section in Fig. 2.
[Fig. 4] Fig. 4 is a perspective view of an ozone generation unit.
[Fig. 5] Fig. 5 is a view of the ozone generation unit when viewed from a transverse direction thereof.
[Fig. 6] Fig. 6 is a view of the ozone generation unit when viewed in an aligning direction thereof.
[Fig. 7] Fig. 7 is an exploded perspective view of the ozone generation unit.
[Fig. 8] Fig. 8 is a perspective view showing a state in which a holder of the ozone generation unit has not been mounted yet.
[Fig. 9] Fig. 9 is a cross-sectional view taken along a line A-A in Fig. 6.
[Fig. 10] Fig. 10 is a perspective view showing a state in which the ozone generation unit is held by a holding portion.
[Fig. 11] Fig. 11 is a block diagram illustrating an electric configuration of the ozone generator.
[Fig. 12] Fig. 12 is an exploded perspective view of a flow path forming portion, a filter frame, a filter, and an intake portion.

[Fig. 13] Fig. 13 is a cross-sectional view illustrating in detail a configuration in the flow path on a cross-section similar to that in Fig. 2.
[Fig. 14] Fig. 14 is a schematic cross-sectional view illustrating an element portion of an ozone sensor.
[Fig. 15] Fig. 15 is a circuit diagram illustrating an electric configuration of the ozone sensor in a simplified manner.
[Fig. 16] Fig. 16 is a bottom view of the ozone generator.
[Fig. 17] Fig. 17 is a bottom view of the ozone generator from which the intake portion has been detached.
[Fig. 18] Fig. 18 is a cross-sectional view of a portion in the vicinity of an inlet and a mounting hole for the filter frame.

MODES FOR CARRYING OUT THE INVENTION

1. First embodiment

1-1. Configuration of ozone generator 100

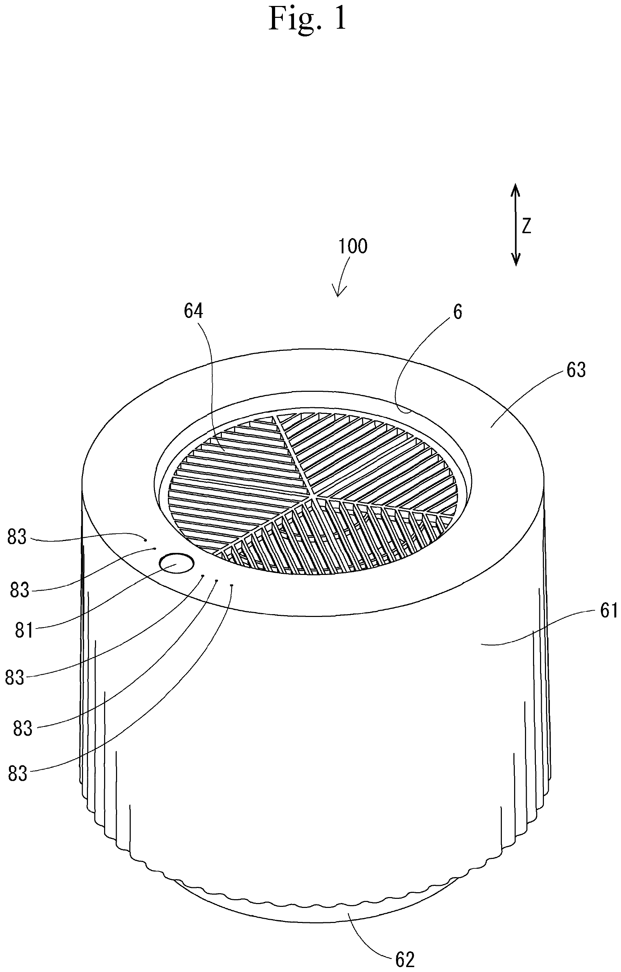

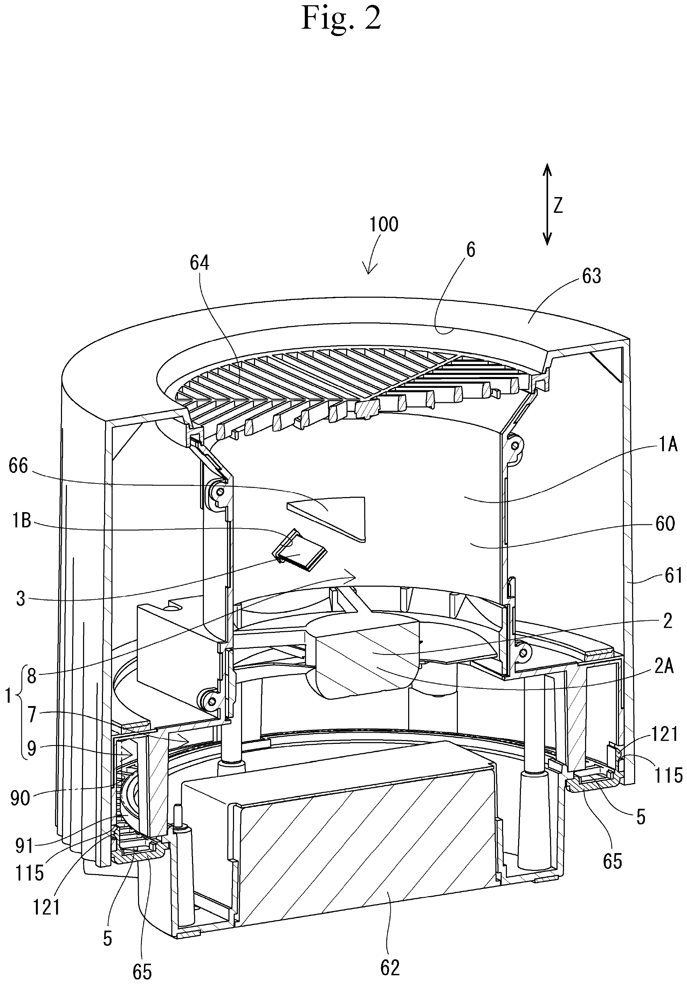

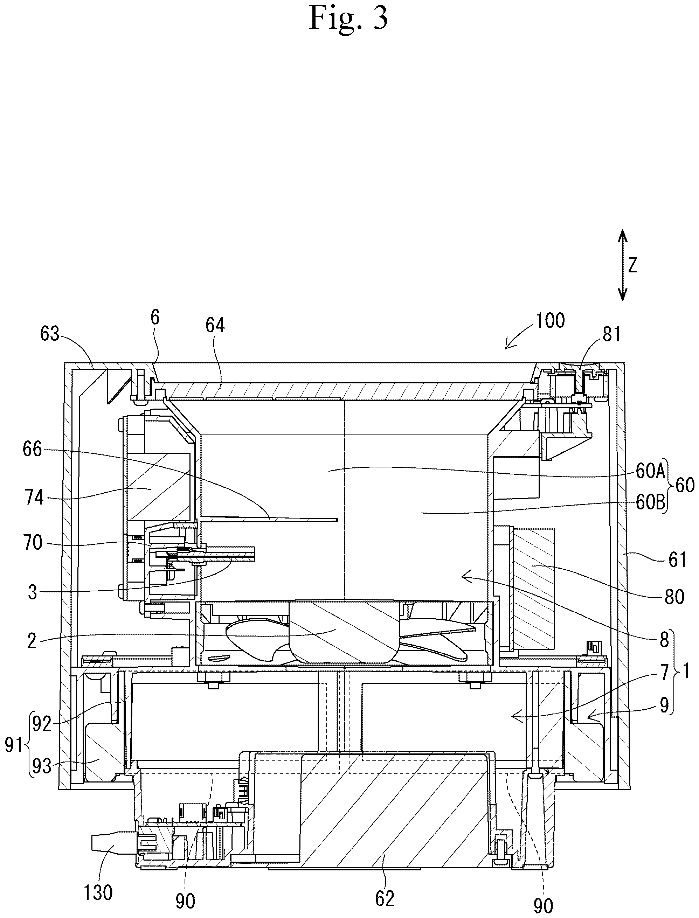

An ozone generator 100 shown in Fig. 1 is a device for taking in air (air containing oxygen) from the outside, generating ozone from oxygen in the air by dielectric barrier discharge, and discharging the ozone to the outside. As shown in Fig. 2 and Fig. 3, the ozone generator 100 has a flow path 1 for gas, a fan 2, an ozone generation unit 3, and an ozone sensor 4.

The flow path 1 has an inlet 5 and an outlet 6. Through the inlet 5, gas (for example, air) outside the ozone generator 100 is taken into the flow path 1. Through the outlet 6, gas in the flow path 1 is discharged to the outside of the ozone generator 100. The flow path 1 allows gas taken in through the inlet 5 to be discharged through the outlet 6.

The flow path 1 extends along a predetermined Z direction (up-down direction in the present embodiment). The inlet 5 is disposed on one end side in the Z direction (lower end side in the present embodiment), and is opened to the one end side in the Z direction (lower side in the present embodiment). The intake direction of the inlet 5 is the direction toward the other end side in the Z direction (upper side in the present embodiment). The outlet 6 is disposed on the other end side in the Z direction (upper end side in the present embodiment), and is opened to the other end side in the Z direction (upper side in the present embodiment). The discharge direction of the outlet 6 is the direction toward the other end side in the Z direction (upper side in the present embodiment).

The inlet 5 is disposed along an annular shape (specifically, round-ring shape) in which the axial direction is the Z direction. The outlet 6 is disposed inward of the annular portion at which the inlet 5 is disposed. The outlet 6 is disposed so as to have a round shape.

The flow path 1 has a guiding flow path 7, an outlet-side flow path 8, and an inlet-side flow path 9. The inlet-side flow path 9 extends from the inlet 5 toward the outlet 6. The inlet-side flow path 9 extends from the inlet 5 toward the other end side in the Z direction (upper side in the present embodiment), and guides gas taken in through the inlet 5 toward the other end side in the Z direction (upper side in the present embodiment). The guiding flow path 7 is connected to a downstream-side end portion of the inlet-side flow path 9, and guides gas that has been taken in through the inlet 5 disposed along the annular shape, inwardly of the inner circumference of the inlet 5. The outlet-side flow path 8 extends from a downstream-side end portion of the guiding flow path 7 to the other end side in the Z direction (upper side in the present embodiment) toward the outlet 6. The downstream-side end portion of the outlet-side flow path 8 is connected to the outlet 6. The outlet-side flow path 8 has an outer shape smaller than the inner circumference of the inlet 5 disposed along the annular shape, and guides the gas that is guided inwardly by the guiding flow path 7 toward the outlet 6 (upper side in the present embodiment), to discharge the gas through the outlet 6. In the description herein, "disposed along an annular shape" means not only disposed annularly in an entirely continuous state but also disposed annularly as a whole in a partially discontinuous state, and can be simply described as "disposed annularly".

The fan 2 is a device for generating airflow (specifically, swirl flow) in the flow path 1, and is an axial flow fan in the present embodiment. The fan 2 performs a blowing operation of sending gas from the inlet 5 side to the outlet 6 side in the flow path 1. The fan 2 has a rotor 2A, a blade portion 2B protruding radially outward from the rotor 2A, and a motor (not shown). The motor is driven when power is supplied, and the fan 2 performs the blowing operation. The fan 2 is disposed in the flow path 1 (specifically, the outlet-side flow path 8). The fan 2 is disposed with the axial direction of the fan 2 directed in the Z direction. The fan 2 rotates with the Z direction as the axial direction. The fan 2 is disposed downstream of the inlet 5 and the ozone sensor 4 described below.

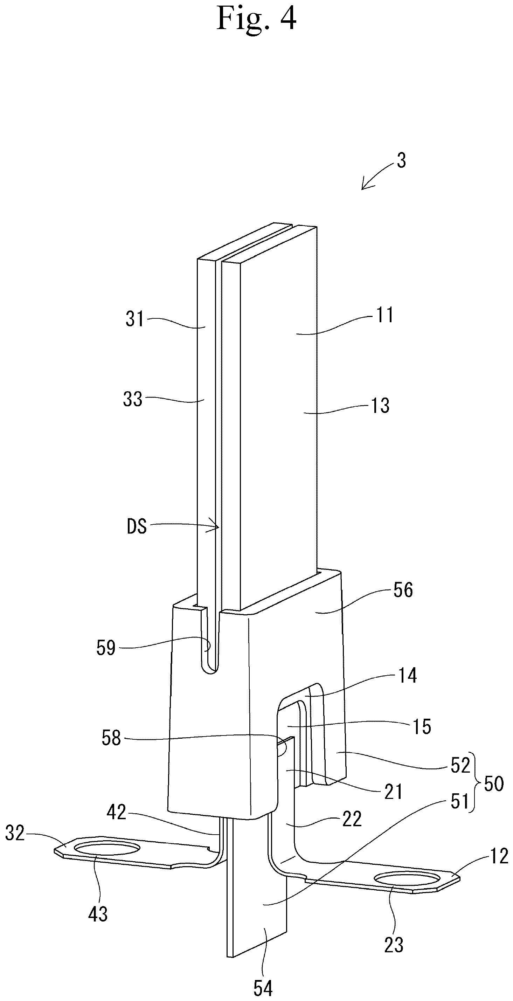

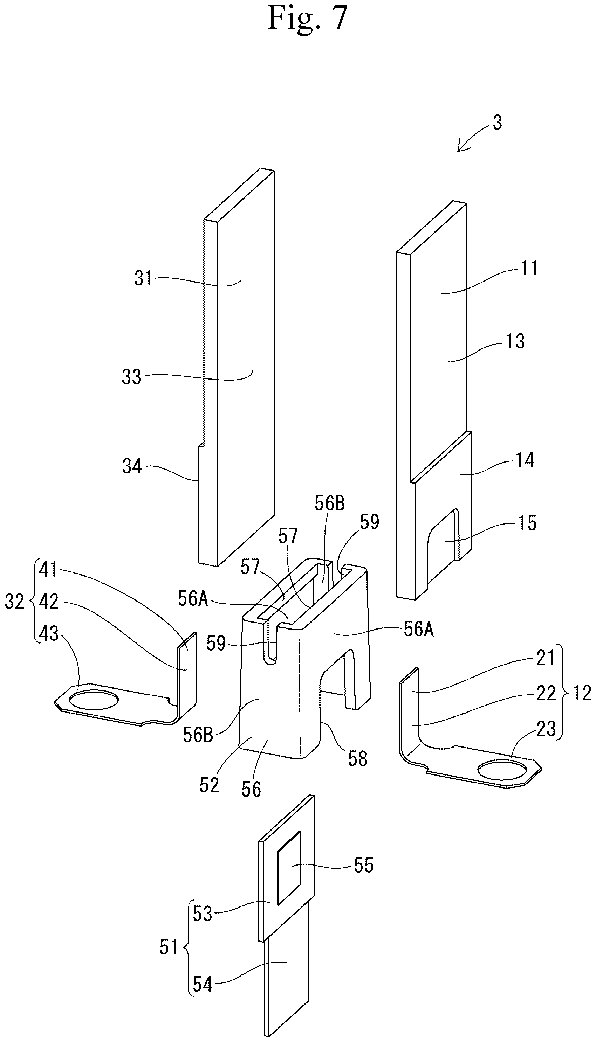

The ozone generation unit 3 causes dielectric barrier discharge by being applied with AC voltage, and generates ozone in the flow path 1 by using, as a material, oxygen in air taken in through the inlet 5. The ozone generation unit 3 is disposed in the flow path 1. As shown in Fig. 4 to Fig. 7, the ozone generation unit 3 has a first electrode 10, a second electrode 30, a first dielectric 11, a second dielectric 31, a first terminal 12, a second terminal 32, and a holding portion 50.

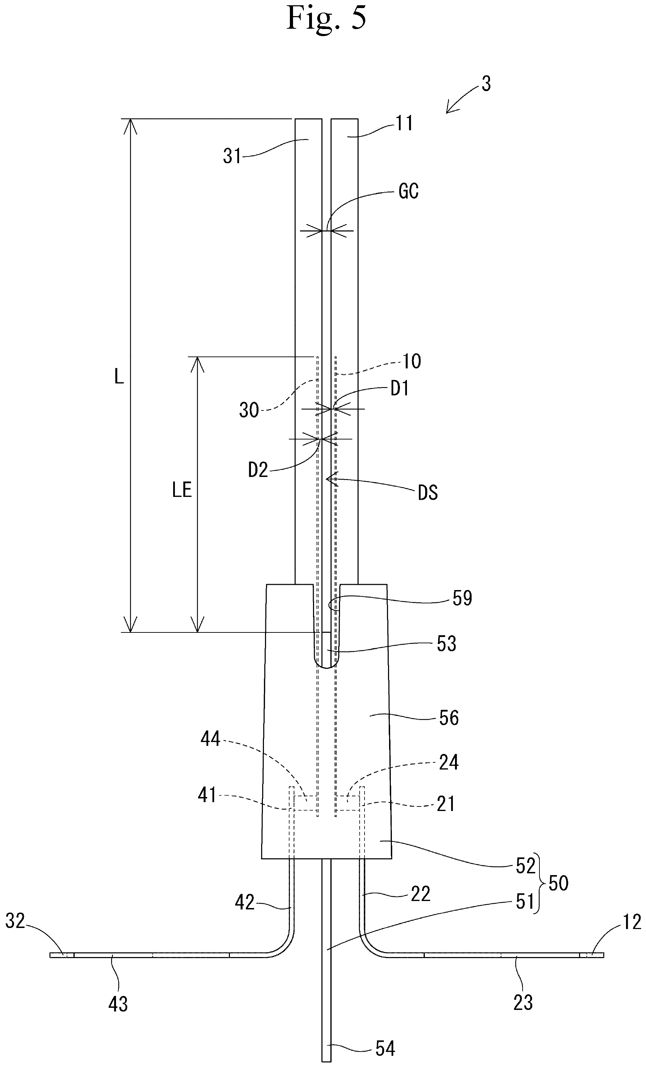

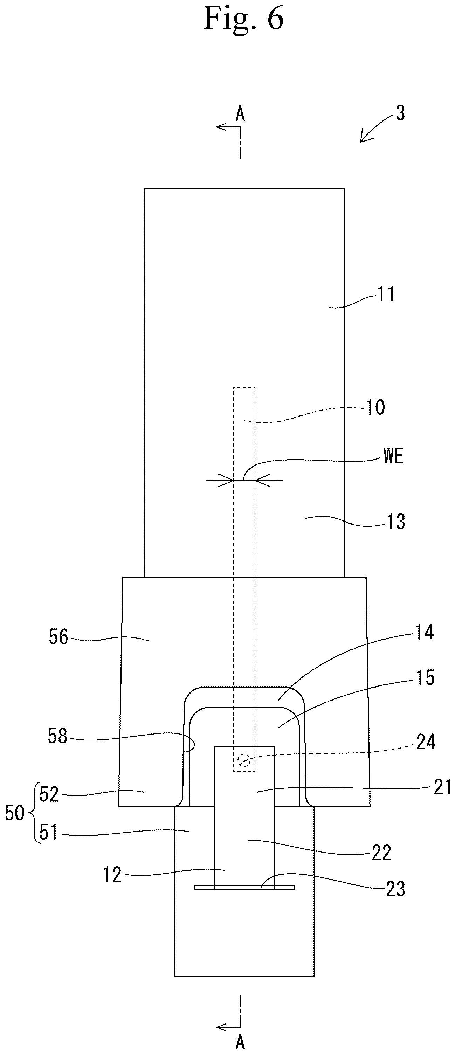

The first electrode 10 and the second electrode 30 are made of metal, and are formed by using tungsten (W) as a material in the present embodiment. The first electrode 10 and the second electrode 30 may be formed by using, for example, molybdenum (Mo), silver (Ag), copper (Cu), or platinum (Pt) as well as tungsten as a material. The first electrode 10 and the second electrode 30 are each formed as a thin metal layer, and elongated in a predetermined direction. The thickness of each of the first electrode 10 and the second electrode 30 (metal layer) is preferably 10 um or greater from the viewpoint of ensuring adhesion strength, and is preferably 50 um or less from the viewpoint of preventing separation due to the excessively great thickness. The width and the length of each of the first electrode 10 and the second electrode 30 are arbitrarily determined according to a required amount of ozone to be generated. A width WE (see Fig. 6) of each of the first electrode 10 and the second electrode 30 is 1 mm. A length of each of the first electrode 10 and the second electrode 30 is set based on a length LE (see Fig. 5) of a portion in which the holding portion 50 is not located between the first electrode 10 and the second electrode 30. The length LE is 10 mm.

The first dielectric 11 and the second dielectric 31 are formed by using alumina ($Al_2O_3$) as a material in the present embodiment. The first dielectric 11 and the second dielectric 31 may be formed by using, as a material, other ceramics such as glass ($SiO_2$), aluminium nitride (AlN), and yttrium oxide ($Y_2O_3$), and mixtures thereof as well as alumina. The first dielectric 11 covers the first electrode 10, and the second dielectric 31 covers the second electrode 30. The first dielectric 11 and the second dielectric 31 are each plate-shaped.

The first dielectric 11 and the second dielectric 31 are aligned in the thickness direction of the first dielectric 11 and the second dielectric 31. That is, the first dielectric 11 and the second dielectric 31 oppose each other in the thickness direction of the first dielectric 11 and the second dielectric 31. A discharge space DS is formed between the first dielectric 11 and the second dielectric 31. The surfaces that oppose each other are each a flat surface, and rectangular. One surface of the surfaces that oppose each other extends along the other surface. One surface of the surfaces that oppose each other may be parallel to the other surface or may not necessarily be parallel thereto. The thickness direction of the first electrode 10 and the second electrode 30 is the same as the thickness direction of the first dielectric 11 and the second dielectric 31. Hereinafter, the direction in which the first dielectric 11 and the second dielectric 31 are aligned is referred to as "aligning direction".

The first electrode 10 is disposed, in the aligning direction, on the second electrode 30 side in the first dielectric 11. The second electrode 30 is disposed, in the aligning direction, on the first electrode 10 side in the second dielectric 31. The first electrode 10 and the second electrode 30 are, for example, printed and disposed on upper surfaces of the thinly formed dielectric layers. By further forming thicker dielectric layers thereon, the first dielectric 11 covering the first electrode 10 and the second dielectric 31 covering the second electrode 30 are produced.

D1 represents a thickness of a portion of the first dielectric 11 that is closer to the discharge space DS than the first electrode 10 is (distance between the surface of the first electrode 10 on the discharge space DS side and the surface of the first dielectric 11 on the discharge space DS side) (see Fig. 5). D2 represents a thickness of a portion of the second dielectric 31 that is closer to the discharge space DS than the second electrode 30 is (distance between the surface of the second electrode 30 on the discharge space DS side and the surface of the second dielectric 31 on the discharge space DS side) (see Fig. 5). In this case, the minimum value of D1+D2 is obtained by the following equation (1).

(Minimum value of D1+D2) = (voltage [kV] applied to the ozone generation unit 3)/(dielectric strength (kV/mm) of material of the first dielectric 11 and the second dielectric 31)     Equation (1)

The dielectric strength of alumina is 15 kV/mm. In a case where the peak value of a high AC voltage is 4.5 kV, the minimum value of D1+D2 is 0.3 mm.

Meanwhile, in a case where D1, D2 is excessively great, loss at the first dielectric 11 and the second dielectric 31 is increased, and power efficiency is lowered. Therefore, the maximum value of D1+D2 is about twice the minimum value of D1+D2. Specifically, D1+D2 is preferably 0.3 mm or more and 0.6 mm or less. That is, each of D1 and D2 is preferably 0.15 mm or more and 0.3 mm or less. In the present embodiment, each of D1 and D2 is 0.15 mm in consideration of facilitating production.

The extending direction (longitudinal direction) of the first electrode 10 and the second electrode 30 is the same as the longitudinal direction of the first dielectric 11 and the second dielectric 31 (hereinafter, simply referred to as "longitudinal direction"). The longitudinal direction corresponds to an example of "orthogonal direction that is orthogonal to the aligning direction of the first dielectric and the second dielectric". Hereinafter, the transverse direction of the first dielectric 11 and the second dielectric 31 is simply referred to as "transverse direction".

The first dielectric 11 has a first dielectric body 13, a first protrusion 14, and a first recess 15. The first dielectric body 13 is plate-shaped and rectangularparallelepiped-shaped. The first dielectric body 13 covers the first electrode 10. The first protrusion 14 protrudes outwardly (toward the side opposite to the second dielectric 31 side) of the first dielectric 11 on one end side in the length direction (longitudinal direction). The first protrusion 14 is formed over the entire region of the first dielectric 11 in the transverse direction. The first protrusion 14 is formed up to the one end of the first dielectric 11 in the length direction (longitudinal direction). The first recess 15 is formed at the surface on the outer side (side opposite to the second dielectric 31 side) of the first dielectric 11 on the one end side in the length direction (longitudinal direction). The first recess 15 is formed by recessing the first protrusion 14. The first recess 15 is opened to the one end of the first dielectric 11 in the length direction (longitudinal direction).

The second dielectric 31 has a second dielectric body 33, a second protrusion 34, and a second recess 35. The second dielectric body 33 is plate-shaped and rectangularparallelepiped-shaped. The second dielectric body 33 covers the second electrode 30. The second dielectric body 33 opposes the first dielectric body 13 to form the discharge space DS between the second dielectric body 33 and the first dielectric body 13. The second protrusion 34 protrudes outwardly (toward the side opposite to the first dielectric 11 side) of the second dielectric 31 on the one end side in the length direction (longitudinal direction). The second protrusion 34 is formed over the entire region of the second dielectric 31 in the transverse direction. The second protrusion 34 is formed up to the one end of the second dielectric 31 in the length direction (longitudinal direction). The second recess 35 is formed at the surface on the outer side (side opposite to the first dielectric 11 side) of the second dielectric 31 on the one end side in the length direction (longitudinal direction). The second recess 35 is formed by recessing the second protrusion 34. The second recess 35 is opened to the one end of the second dielectric 31 in the length direction (longitudinal direction).

Considering that the dielectric strength of air is about 3.0 kV/mm, a gap GC between the dielectrics (see Fig. 5) which is a distance between the first dielectric 11 (specifically, the first dielectric body 13) and the second dielectric 31 (specifically, the second dielectric body 33) needs to be less than 1.5 mm for discharging in a case where a peak value of AC voltage applied to the ozone generation unit 3 is 4.5 kV. However, in order to elongate a discharge time and stably maintain the discharging, the gap GC is preferably one-third of that or less, that is, 0.5 mm or less. Meanwhile, if the gap GC between the dielectrics is excessively small, air to be supplied is inefficient, so that an amount of generated ozone is reduced. Therefore, the gap GC between the dielectrics is preferably 0.2 mm or more. For example, the gap GC between the dielectrics is preferably 0.37 mm. In a case where the opposing surfaces of the first dielectric 11 and the second dielectric 31 are not parallel to each other, the gap GC between the dielectrics is determined with reference to the positions of the tip ends (the other ends in the length direction (longitudinal direction)) of the first electrode 10 and the second electrode 30.

A natural frequency Fn [Hz] of each of the first dielectric 11 and the second dielectric 31 is 200 Hz or higher in a structure in which the first dielectric 11 and the second dielectric 31 are each held at one end thereof. The natural frequency Fn [Hz] may be derived from an experimental result, or may be obtained by an arithmetic expression. In a case where the natural frequency Fn [Hz] is obtained by an arithmetic expression, the natural frequency Fn [Hz] can be obtained, for example, by the following equation (A).

[Math. 1]

$$Fn = \frac{(Kn)^2}{2\pi} \sqrt{\frac{EI}{\rho AL^4}} \quad \cdots \text{Equation (A)}$$

Kn represents a constant, and is 1.875 in a structure in which the first dielectric 11 and the second dielectric 31 are each held at one end thereof. E [Pa] represents a Young's modulus of the first dielectric 11 and the second dielectric 31. E [Pa] is about 280 GPa in a case where the first dielectric 11 and the second dielectric 31 are made of alumina. I [m4] represents second moment of area of the first dielectric 11 and the second dielectric 31. $\rho$ [kg/m3] represents a density of the first dielectric 11 and the second dielectric 31. A [m2] represents a cross-sectional area of the first dielectric 11 and the second dielectric 31. L [m] represents a length from a fixed end of the first dielectric 11 and the second dielectric 31 held by the holding portion 50, to a free end (see Fig. 5).

L needs to be longer than the length LE of each of the first electrode 10 and the second electrode 30. Meanwhile, if L is excessively long, the natural frequency Fn [Hz] becomes low. Therefore, in the present embodiment, L is 21.5 mm. In this case, the natural frequency Fn [Hz] is 3500 Hz, which is significantly higher than 200 Hz. In a case where the thickness of each of the first dielectric 11 and the second dielectric 31 is 1.15 mm, the natural frequency Fn [Hz] is 200 Hz or higher if L is 90 mm or less. Also, if the thickness of each of the first dielectric 11 and the second dielectric 31 is made greater, the natural frequency Fn [Hz] can be 200 Hz, even if L is longer.

The first electrode 10 and the second electrode 30 have the same size and shape, and are disposed with a plane-symmetrical positional relationship. The first dielectric 11 and the second dielectric 31 have the same size and shape, and are disposed with a plane-symmetrical positional relationship.

The first terminal 12 and the second terminal 32 are each made of metal and plate-shaped. The first terminal 12 is disposed in the first recess 15, and the second terminal 32 is disposed in the second recess 35. The first terminal 12 is electrically connected to the first electrode 10, and the second terminal 32 is electrically connected to the second electrode 30. The first terminal 12 and the second terminal 32 are each L-shaped when viewed from the transverse direction.

The first terminal 12 has a first connection portion 21, a first protrusion 22, and a third connection portion 23.

The first connection portion 21 is electrically connected to the first electrode 10 via a first conductive portion 24 disposed in the first dielectric 11, as shown in Fig. 5 and Fig. 6. In the present embodiment, the first conductive portion 24 is a via hole formed in the first dielectric 11. The first conductive portion 24 extends from the first electrode 10 to the surface on the outer side (side opposite to the second dielectric 31 side) of the first dielectric 11. The first conductive portion 24 is exposed at the surface on the outer side (side opposite to the second dielectric 31 side) of the first dielectric 11, and a land is formed at the exposed portion of the first conductive portion 24. The first connection portion 21 is brazed to the land. Thus, the first terminal 12 is electrically connected to the first electrode 10. The first protrusion 22 is continuous with one end of the first connection portion 21, and protrudes further to the one end side than the end portion of the first dielectric 11. The third connection portion 23 is bent from the end (end portion on the one end side) of the first protrusion 22 and extends in the aligning direction.

The second terminal 32 has a second connection portion 41, a second protrusion 42, and a fourth connection portion 43. The second connection portion 41 is electrically connected to the second electrode 30 via a second conductive portion 44 disposed in the second dielectric 31. In the present embodiment, the second conductive portion 44 is a via hole formed in the second dielectric 31. The second connection portion 41 is connected to the second electrode 30 in a manner similar to the above-described manner in which the first connection portion 21 and the first electrode 10 are connected to each other. The second protrusion 42 is continuous with one end of the second connection portion 41, and protrudes further to the one end side than the end portion of the second dielectric 31. The fourth connection portion 43 is bent from the end (end portion on the one end side) of the second protrusion 42 and extends in the aligning direction. The third connection portion 23 and the fourth connection portion 43 extend in opposite directions.

The holding portion 50 holds the first dielectric 11 and the second dielectric 31. The holding portion 50 holds the first dielectric 11 and the second dielectric 31 only at the one end side in the length direction (longitudinal direction). That is, the holding portion 50 holds each of the first dielectric 11 and the second dielectric 31 only at the one end on the same side. The holding portion 50 has a Young's modulus that is lower than those of the first dielectric 11 and the second dielectric 31. The holding portion 50 is formed by using resin (for example, polycarbonate (PC), ABS, PVC, PP, or the like) as a material. The Young's modulus of a resin material such as PC is about 1 to 2.5 GPa, and is very low as compared with alumina having a Young's modulus of 280 GPa. Therefore, vibration of the first dielectric 11 and the second dielectric 31 formed of alumina is easily absorbed by the holding portion 50. The holding portion 50 has a spacer 51 and a holder 52.

The spacer 51 is disposed between the first dielectric 11 and the second dielectric 31 at the one end side in the length direction (longitudinal direction), and allows the discharge space DS to be formed between the first dielectric 11 and the second dielectric 31 at the other end side in the length direction (longitudinal direction). The spacer 51 is plate-shaped. The spacer 51 is disposed with the thickness direction thereof directed in the aligning direction of the first dielectric 11 and the second dielectric 31. The spacer 51 has a spacer portion 53 disposed between the first dielectric 11 and the second dielectric 31, and an extending portion 54 which extends from the spacer portion 53 toward the one end side in the length direction (longitudinal direction) and is disposed between the first protrusion 22 and the second protrusion 42.

The spacer portion 53 is plate-shaped. In the transverse direction, the spacer portion 53 can be stored in a range of the first dielectric 11 and the second dielectric 31. One end of the spacer portion 53 in the length direction (longitudinal direction) is disposed closer to the other end side than one end of each of the first dielectric 11 and the second dielectric 31 in the length direction (longitudinal direction). The other end of the spacer portion 53 in the length direction (longitudinal direction) is disposed closer to the one end side than the other end of each of the first protrusion 14 and the second protrusion 34.

The extending portion 54 is plate-shaped. The extending portion 54 has a thickness less than the thickness of the spacer portion 53. The thickness of the extending portion 54 may not necessarily be less than the thickness of the spacer portion 53, and may be, for example, the same as the thickness of the spacer portion 53. In the transverse direction, the extending portion 54 extends outwardly of the end portions on both sides of each of the first terminal 12 and the second terminal 32. In the longitudinal direction, the extending portion 54 extends closer to the one end side than the end portion, on the one end side, of each of the first terminal 12 and the second terminal 32.

The ozone generation unit 3 has a double-sided tape 55 for adhering the first dielectric 11 and the second dielectric 31 to the spacer 51. Each of the first dielectric 11 and the second dielectric 31 is adhered to the spacer portion 53 of the spacer 51 through the double-sided tape 55.

The holder 52 is a member for holding the first dielectric 11 and the second dielectric 31 holding the spacer 51 therebetween. The holder 52 has a ring shape (specifically, rectangular-tube-like shape), and is disposed so as to surround the outer circumference of the first dielectric 11 and the second dielectric 31 holding the spacer 51 therebetween. The holder 52 may have a round-ring shape or may have a shape other than a round-ring shape. The holder 52 has a holder body 56, a lock-engagement portion 57, a first cut portion 58, and a second cut portion 59.

The holder body 56 has a ring shape (specifically, rectangular-tube-like shape). The holder body 56 may have a round-ring shape or may have a shape other than a round-ring shape. The holder body 56 has a pair of first wall portions 56A disposed on both sides in the aligning direction, and a pair of second wall portions 56B disposed on both sides in the transverse direction.

The lock-engagement portion 57 is formed so as to protrude inwardly from the inner surface of the holder body 56 on the other end side in the length direction (longitudinal direction). The lock-engagement portion 57 protrudes from each of the inner surfaces of the pair of first wall portions 56A. The lock-engagement portion 57 is formed over the entire region of each of the first wall portions 56A in the transverse direction.

The first cut portion 58 is formed by cutting so as to expose the first terminal 12 and the second terminal 32. The first cut portion 58 is formed by cutting the end portions of the pair of first wall portions 56A on the one end side in the length direction (longitudinal direction).

The second cut portion 59 is formed by cutting so as to expose the discharge space DS. The second cut portion 59 is formed by cutting the end portions of the pair of second wall portions 56B on the other end side in the length direction (longitudinal direction). One end of the second cut portion 59 in the length direction (longitudinal direction) is disposed closer to the other end side than the other end of the first cut portion 58 in the length direction (longitudinal direction). The width (space in the aligning direction) of the second cut portion 59 is preferably greater than the gap GC between the dielectrics.

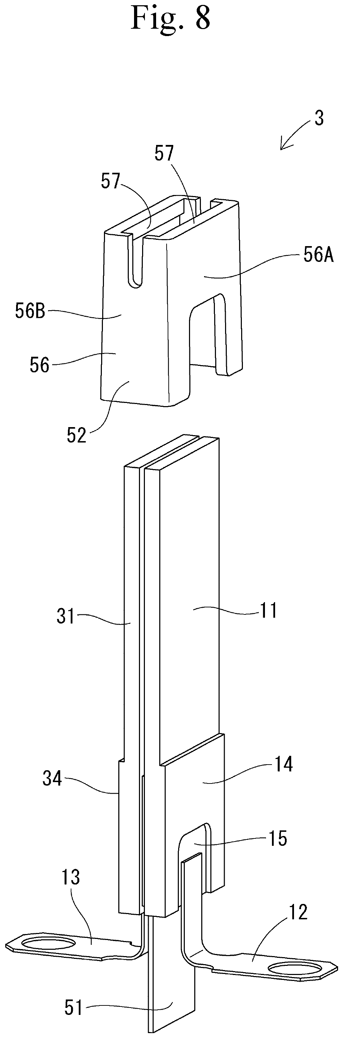

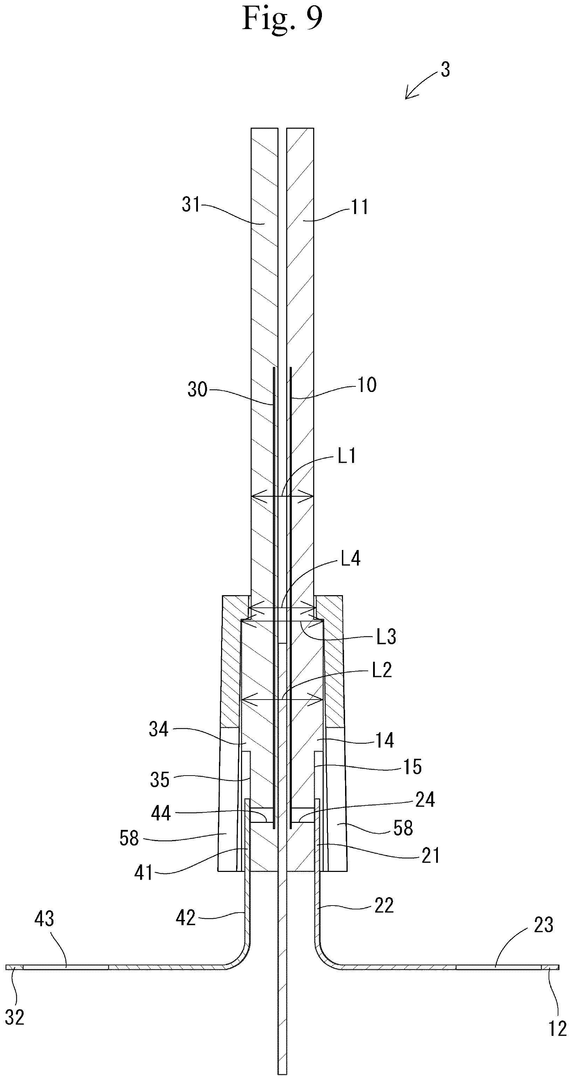

As shown in Fig. 8, the holder 52 is inserted onto the first dielectric 11 and the second dielectric 31 holding the spacer 51 therebetween from the other end side in the length direction (longitudinal direction). The holder 52 is positioned by bringing the lock-engagement portion 57 into contact with the end portion of each of the first protrusion 14 of the first dielectric 11 and the second protrusion 34 of the second dielectric 31 in the length direction (longitudinal direction). As shown in Fig. 9, when, in the aligning direction, L1 represents a distance between the outer surfaces of the first dielectric body 13 and the second dielectric body 33 holding the spacer 51 therebetween, L2 represents a distance between the outer surfaces of the first protrusion 14 and the second protrusion 34 holding the spacer 51 therebetween, L3 represents a minimum distance between inner surfaces of the pair of the second wall portions 56B of the holder 52, and L4 represents a distance between inner surfaces of the pair of the lock-engagement portions 57 of the holder 52, the following expressions (2) and (3) are satisfied.

$$L1 \leq L4 \quad \cdots \text{Expression (2)}$$

$$L4 < L2 \leq L3 \quad \cdots \text{Expression (3)}$$

As shown in Fig. 2 and Fig. 3, the ozone generator 100 has a flow path forming portion 60, a circumferential wall portion 61, a bottom portion 62, a ceiling portion 63, a finger guard 64, an intake portion 65, and a diffusion plate 66.

The flow path forming portion 60 is a portion that forms the flow path 1. The flow path forming portion 60 is structured to be segmented into a plurality (two in the present embodiment) of segmented portions in the circumferential direction. Specifically, the flow path forming portion 60 is segmented into a first segmented portion 60A and a second segmented portion 60B in the circumferential direction, and is formed by connecting the first segmented portion 60A and the second segmented portion 60B to each other.

The circumferential wall portion 61 has an annular shape (specifically, cylindrical shape, more specifically, round-cylinder-shape), and is formed so as to surround the outer circumference of the flow path forming portion 60 and the flow path 1. The diameter (outer diameter of the circumferential wall portion 61) of the outer circumference of the ozone generator 100 is 225 mm, and the height of the ozone generator 100 is 204 mm.

The bottom portion 62 is a portion placed on a placement surface. The bottom portion 62 holds the flow path forming portion 60 placed thereon. The bottom portion 62 is configured to fit inside the inlet 5 which is disposed annularly. The bottom portion 62 has an outer shape smaller than the inner circumference of the circumferential wall portion 61.

The ceiling portion 63 is disposed on the other end side in the Z direction in the ozone generator 100, and has an annular shape in which the axial direction is the Z direction. The outlet 6 is formed inside the ceiling portion 63. The ceiling portion 63 has an outer circumference connected to the end portion (upper end portion in the present embodiment) of the circumferential wall portion 61 on the other end side, and is formed integrally with the circumferential wall portion 61. The circumferential wall portion 61 and the ceiling portion 63 are disposed above the flow path forming portion 60 with the finger guard 64 interposed therebetween, and are held by the flow path forming portion 60. The circumferential wall portion 61 is held so as to be spaced from the placement surface.

The finger guard 64 is a planar (disk-shaped in the present embodiment) portion having a plurality of through holes. The through holes are each slit-shaped. The finger guard 64 has a function of preventing a foreign object (for example, a finger or the like) from entering from the outside while allowing gas in the flow path 1 to be discharged. The finger guard 64 is formed as a member separate from the flow path forming portion 60 and the ceiling portion 63. The finger guard 64 is disposed downstream of the diffusion plate 66.

The intake portion 65 is a portion that forms the inlet 5, and has an annular shape. The intake portion 65 is disposed between the inner circumference of the circumferential wall portion 61 on the lower end side and the outer circumference of the bottom portion 62 on the upper end side, and is lock-engaged with respect to the flow path forming portion 60. The intake portion 65 has a plurality of the inlets 5. The plurality of the inlets 5 is arranged annularly along the annular intake portion 65. Each inlet 5 is elongated in the radial direction.

The diffusion plate 66 is for diffusing ozone generated by the ozone generation unit 3 in the flow path 1. The diffusion plate 66 is disposed downstream of the ozone generation unit 3 in the flow path 1. The diffusion plate 66 is formed so as to protrude inwardly from a wall surface 1A of the flow path 1. The diffusion plate 66 protrudes from a part, in the circumferential direction, of the wall surface 1A. The farther the diffusion plate 66 is away from the wall surface 1A, the smaller the width of the diffusion plate 66 is. The diffusion plate 66 is sectorshaped. The diffusion plate 66 is positioned so as to overlap the ozone generation unit 3 when viewed from the other end side in the Z direction. The diffusion plate 66 is formed integrally with the flow path forming portion 60 (specifically, the first segmented portion 60A).

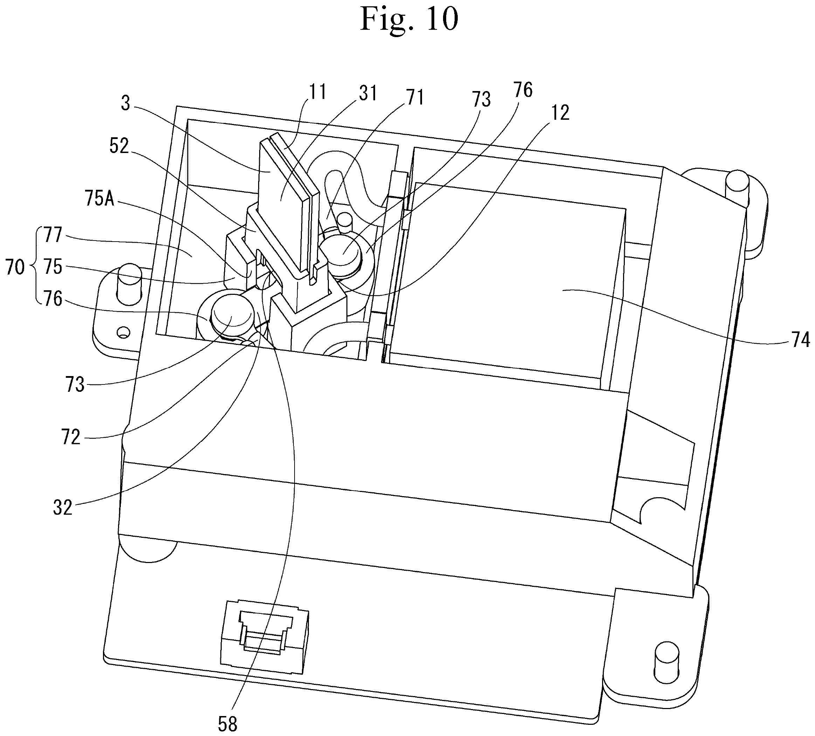

As shown in Fig. 3 and Fig. 10, the ozone generator 100 has a holding portion 70, a first counterpart terminal 71, a second counterpart terminal 72, screws 73, and an AC power supply 74.

The holding portion 70 is a portion for holding the ozone generation unit 3. The holding portion 70 has a first storage portion 75, terminal fixing portions 76, and a second storage portion 77. The first storage portion 75 has a bottom surface, and a surrounding portion that protrudes from the bottom surface and surrounds the outer peripheral portion of the holder 52. In the first storage portion 75, one end side portion of the holder 52 in the length direction (longitudinal direction) is stored. At least a part of the holder 52 protrudes from the opening end of the first storage portion 75. The first storage portion 75 has a cut groove 75A into which the first terminal 12 and the second terminal 32 of the ozone generation unit 3 fit. The terminal fixing portion 76 is provided so as to correspond to each of the first terminal 12 and the second terminal 32. The terminal fixing portion 76 has a female screw. The third connection portion 23 of the first terminal 12 is screwed together with the first counterpart terminal 71 to one of the terminal fixing portions 76 by the screw 73. The fourth connection portion 43 of the second terminal 32 is screwed together with the second counterpart terminal 72 to the other of the terminal fixing portions 76 by the screw 73. The first counterpart terminal 71 and the second counterpart terminal 72 are each electrically connected to the AC power supply 74.

In the second storage portion 77, one end side portion, in the length direction (longitudinal direction), of the ozone generation unit 3 stored, and at least the entirety of the first terminal 12 and the second terminal 32 are stored. The internal portion of the second storage portion 77 is resin-molded so as to embed at least the entirety of the first terminal 12 and the second terminal 32. At least a part of the holder 52 (specifically, at least a portion closer to the other end side than the one end of the second cut portion 59 in the length direction (longitudinal direction)) protrudes from the molded resin.

The holding portion 70 is fixed with respect to the outer surface of the flow path forming portion 60. As shown in Fig. 3, the holding portion 70 is disposed outside the wall surface 1A of the flow path 1, and holds the holding portion 50 of the ozone generation unit 3 outside the wall surface 1A. An opening 1B through which the ozone generation unit 3 protrudes inward is formed in the wall surface 1A of the flow path 1. The first dielectric 11 and the second dielectric 31 of the ozone generation unit 3 are disposed so as to protrude from the opening 1B to the inside of the wall surface 1A. At least a part of the first electrode 10 and at least a part of the second electrode 30 are disposed inward of the wall surface 1A. The holder 52 is also disposed so as to protrude from the opening 1B to the inside of the wall surface 1A.

The AC power supply 74 has a transformer, and can supply AC power. The AC power supply 74 generates predetermined AC power based on power supplied from a commercial power supply outside the ozone generator 100, and supplies the AC power to the ozone generation unit 3 and the like.

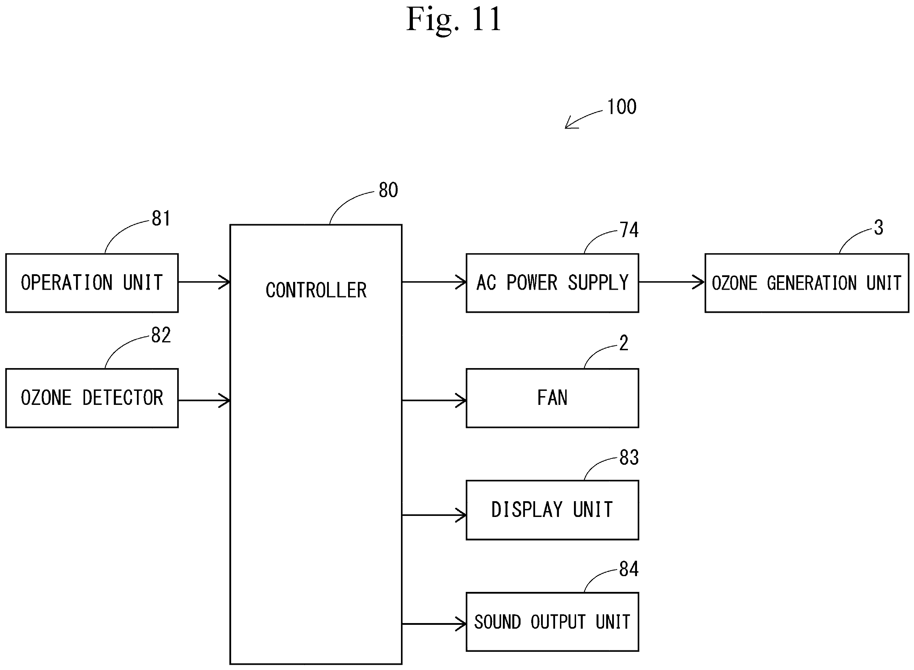

As shown in Fig. 11, the ozone generator 100 has a controller 80, an operation unit 81, an ozone detector 82, a display unit 83, and a sound output unit 84. The controller 80 controls an operation of the ozone generator 100. The controller 80 is configured mainly by a microcomputer, and has a CPU, a ROM, a RAM, a drive circuit, and the like.

The operation unit 81 is, for example, a switch that switches between an on-state and an off-state by being pressed, and is, for example, a tact switch. A signal indicating a result of an operation on the operation unit 81 is inputted to the controller 80. The ozone detector 82 detects an ozone concentration in air outside the ozone generator 100. A signal indicating a detection value of the ozone detector 82 is inputted to the controller 80.

The controller 80 can control an operation of the ozone generation unit 3 via the AC power supply 74. The controller 80 controls AC voltage to be applied to the ozone generation unit 3, and can thus adjust an amount of ozone to be generated by the ozone generation unit 3. The controller 80 can adjust an amount of generated ozone based on a result of an operation on the operation unit 81. The controller 80 can perform feedback-control for an operation of the ozone generation unit 3 based on the ozone concentration detected by the ozone detector 82 such that the ozone concentration approaches a target value.

The controller 80 can control an operation of the fan 2. The controller 80 provides the fan 2 with a PWM signal to perform PWM-control for the fan 2. Thus, the controller 80 can adjust an airflow amount.

The controller 80 can control an operation of the display unit 83. The display unit 83 is, for example, a LED lamp. The display unit 83 displays an on/off state of the power supply, an operation state of the fan 2, an ozone concentration in the outside, and the like, by the lighting state of the LED.

The controller 80 can control an operation of the sound output unit 84. The sound output unit 84 outputs a sound, and is, for example, a buzzer. The sound output unit 84 outputs a warning sound in a case where, for example, the ozone generator 100 becomes abnormal.

1-2. Configuration of filter 90

As shown in Fig. 2, Fig. 3, and Fig. 12, the ozone generator 100 has a filter 90 and a filter frame 91.

The filter 90 is disposed in the guiding flow path 7. The filter 90 is, for example, an HEPA (high efficiency particulate air) filter, a medium efficiency filter, or the like. The filter 90 has an annular shape (specifically, round-ring shape). The filter 90 is segmented into a plurality (four in the present embodiment) of segmented filters 90A in the circumferential direction.

The filter frame 91 has a frame body 92 and a tab 93. The frame body 92 has an annular shape (specifically, round-ring shape). The frame body 92 is segmented into a plurality (two in the present embodiment) of segmented frames 92A in the circumferential direction. The frame body 92 has a mounting hole 94 in which the filter 90 is mounted. A plurality (four in the present embodiment) of the mounting holes 94 is disposed along the circumferential direction of the filter frame 91. The segmented filters 90A are each mounted in the corresponding mounting hole 94. Thus, the filter 90 is mounted in the mounting holes 94. The tab 93 protrudes outwardly from the outer circumferential surface of the frame body 92. The tab 93 has a plate shape with the circumferential direction as the thickness direction. The tab 93 is disposed at the center portion, in the circumferential direction, of the segmented frame 92A. The tab 93 is disposed closer to one side of the frame body 92 in the axial direction. The tab 93 protrudes from one end of the frame body 92 in the axial direction of the frame body 92.

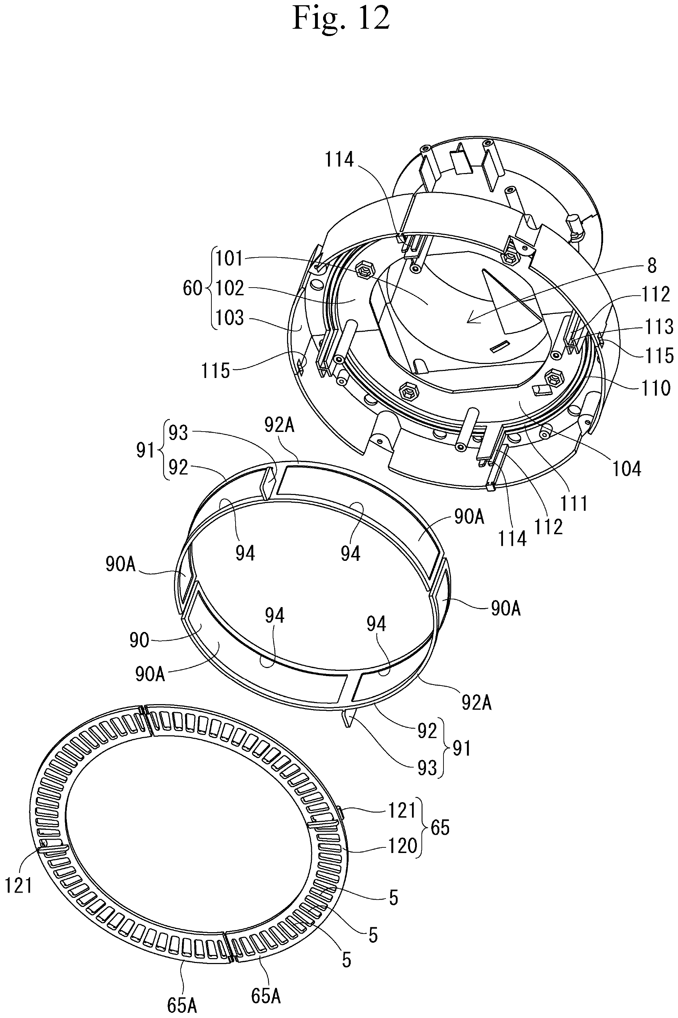

As shown in Fig. 12, the flow path forming portion 60 described above has a cylindrical (specifically, round-cylinder-shaped) first component 101 which forms the outlet-side flow path 8, a second component 102 which extends radially outward from the one end side of the first component 101 in the Z direction, and an annular third component 103 that extends from the end portion of the outer circumferential side of the second component 102 toward the one end side in the Z direction. The diameter of the first component 101 is less than the diameter of the second component 102. Particularly, the diameter of the first component 101 is less than the outer diameter (diameter of the outer circumferential edge) of the second component 102. The outlet 6 is disposed on the other end side, in the Z direction, of the first component 101.

The second component 102 has a component surface 104 which has an annular shape and faces the one end side in the Z direction. The component surface 104 forms a part of the guiding flow path 7. The second component 102 further has a mounting portion 110 to which the filter frame 91 is mounted. The mounting portion 110 is disposed on the component face 104. The fan 2 is disposed in the second component 102.

A height (height in the Z-axis direction) of the first component 101 is greater than a height (height in the Z-axis direction) of the second component 102 and a height (height in the Z-axis direction) of the third component. More specifically, the height (height in the Z-axis direction) of the first component 101 is preferably twice or greater than each of the height (height in the Z-axis direction) of the second component 102 and the height (height in the Z-axis direction) of the third component. The height (height in the Z-axis direction) of the first component 101 is greater than each of the size of the second component 102 in the radial direction and the size in the radial direction. More specifically, the height (height in the Z-axis direction) of the first component 101 is preferably twice or greater than each of the size of the second component 102 in the radial direction and the size in the radial direction.

The intake portion 65 is disposed on the one end side of the filter frame 91 in the Z direction. The intake portion 65 (more specifically, the inlet 5) is disposed radially outward of the first component 101. As shown in Fig. 12, the intake portion 65 has an annular shape (specifically, round-ring shape). The intake portion 65 has a plate shape with the axial direction as the thickness direction. The inlet 5 is formed in the intake portion 65 so as to penetrate therethrough in the axial direction. The inlets 5 are arranged at equal intervals along the circumferential direction of the intake portion 65. The inlet 5 has the length in the radial direction of the intake portion 65 greater than the length in the circumferential direction of the intake portion 65. The intake portion 65 (more specifically, the inlet 5) is disposed radially outward of the outlet 6.

1-3. Structure for mounting the filter 90

Next, a structure for mounting the filter 90 will be described.

The mounting portion 110 has a mounting groove 111, a guide groove 112, a partition 113, and a tab storage groove 114.

The mounting groove 111 is arranged on the component surface 104, and is formed in an annular shape (specifically, round-ring shape) so as to surround the outer circumference of the first component 101 (the outlet-side flow path 8). The mounting groove 111 is opened to the one end side in the Z direction. The filter frame 91 is mounted to the mounting groove 111.

The guide groove 112 is formed by extending a part, in the circumferential direction, of the mounting groove 111 toward the one end side in the Z direction. A plurality (four in the present embodiment) of the guide grooves 112 is arranged at equal intervals along the circumferential direction of the mounting groove 111. The partitions 113 are portions for partitioning the segmented frames 92A, and are disposed in the mounting groove 111 and the guide grooves 112. The tab storage groove 114 is a portion in which the tab 93 of the filter frame 91 is stored, and the tab storage groove 114 extends radially outward from the guide groove 112.

The filter 90 is firstly mounted to the filter frame 91 in order to mount the filter frame 91 to the mounting portion 110. The filter frame 91 is moved to the other end side in the Z direction along the guide groove 112 in such an orientation that the tab 93 is disposed at the one end side in the Z direction, and mounted to the mounting groove 111. Specifically, the segmented frame 92A is individually mounted to a portion partitioned by the partition 113. The filter frame 91 fits into the mounting groove 111, whereby mounting to the mounting portion 110 is completed. The filter frame 91 is allowed to be moved toward the one end side in the Z direction since the filter frame 91 is not lock-engaged with the mounting portion 110. That is, the filter frame 91 is allowed to be removed from the mounting portion 110.

The third component 103 has an attachment portion 115. The attachment portion 115 is formed as a lock-engagement groove. By attaching the intake portion 65 described above to the attachment portion 115, the intake portion 65 is disposed at the one end side of the filter frame 91 in the Z direction, so that the filter frame 91 is prevented from being removed from the mounting portion 110.

As shown in Fig. 12, the intake portion 65 has an intake portion body 120 and an attaching portion 121.

The intake portion body 120 has an annular shape (specifically, round-ring shape). The intake portion body 120 is segmented into a plurality (two in the present embodiment) of segmented intake portions 120A in the circumferential direction. The intake portion body 120 has a plate shape with the axial direction of the intake portion body 120 as the thickness direction. The inlet 5 is formed so as to penetrate in the axial direction of the intake portion body 120. The inlets 5 are arranged at equal intervals along the circumferential direction of the intake portion body 120. In the inlet 5, the length in the radial direction of the intake portion body 120 is greater than the length in the circumferential direction of the intake portion body 120.

The attaching portion 121 is attached to the attachment portion 115 of the flow path forming portion 60. As shown in Fig. 2, the attaching portion 121 is a cantilever-like lock-engagement piece extending toward the other end side in the Z direction. When the intake portion 65 is pressed toward the other end side in the Z direction against the flow path forming portion 60, the leading end side of the lock-engagement piece as the attaching portion 121 is deformed. When the intake portion 65 is further pressed, the attaching portion 121 is lock-engaged with the lock-engagement groove as the attachment portion 115 by its own elastic force. Thus, the intake portion 65 is attached to the attachment portion 115 of the flow path forming portion 60. The intake portion 65 attached to the attachment portion 115 prevents the filter frame 91 from being removed from the mounting portion 110.

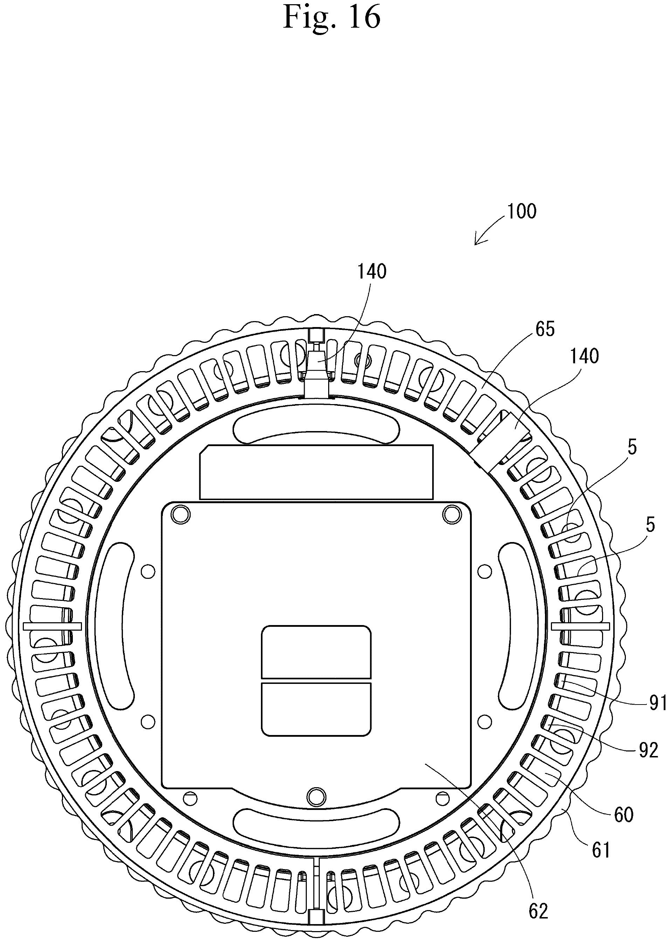

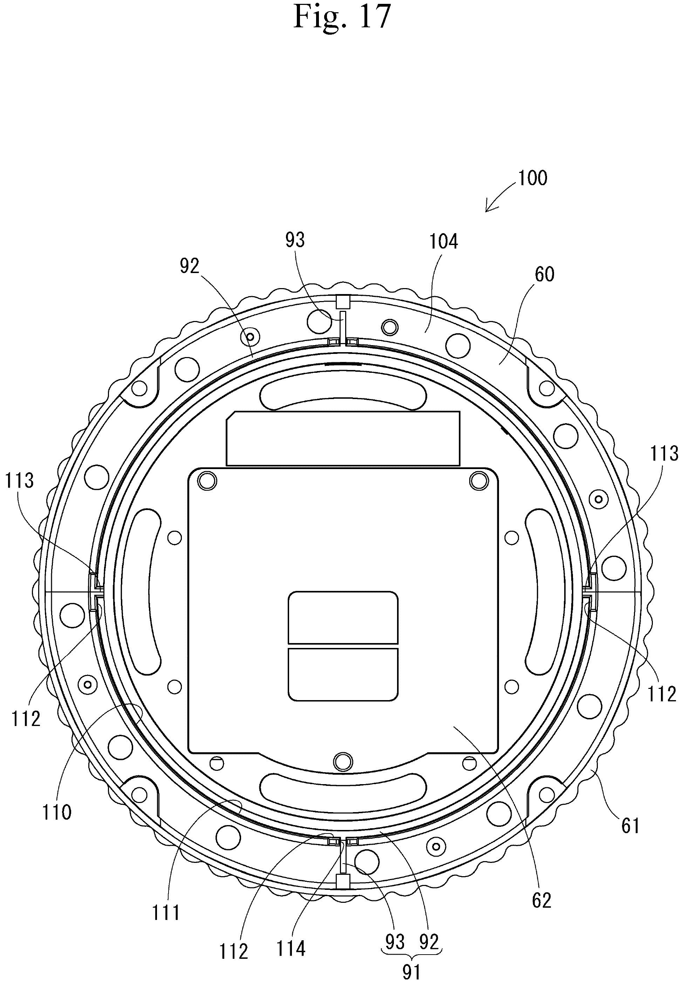

When replacing the filter 90, from the state shown in Fig. 16, the intake portion 65 and a plug 140 are removed, and the ozone generator 100 becomes in the state shown in Fig. 17. The intake portion 65 is removed by being pulled toward the one end side in the Z direction. In the state shown in Fig. 17, the filter frame 91 is exposed at the one end side in the Z direction. The bottom portion 62 is adjacently disposed at the inner circumference of the frame body 92 of the filter frame 91. Therefore, it is difficult to pinch the frame body 92. However, the tab 93 is arranged at the outer circumference of the frame body 92 so as to protrude radially outward. Thus, an operator can easily remove the filter frame 91 by pinching the tab 93, and replace the filter 90.

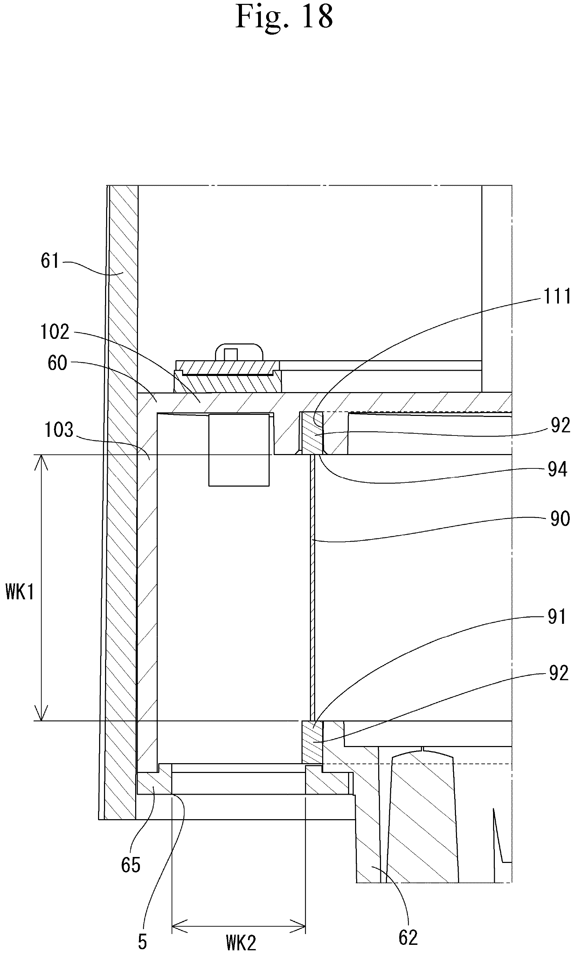

As shown in Fig. 18, an opening width WK1 of the mounting hole 94 in the axial direction (the Z direction) of the inlet 5 disposed along an annular shape is greater than an opening width WK2 of the inlet 5 in the direction orthogonal to the axial direction (the Z direction).

1-4. Configuration of ozone sensor 4

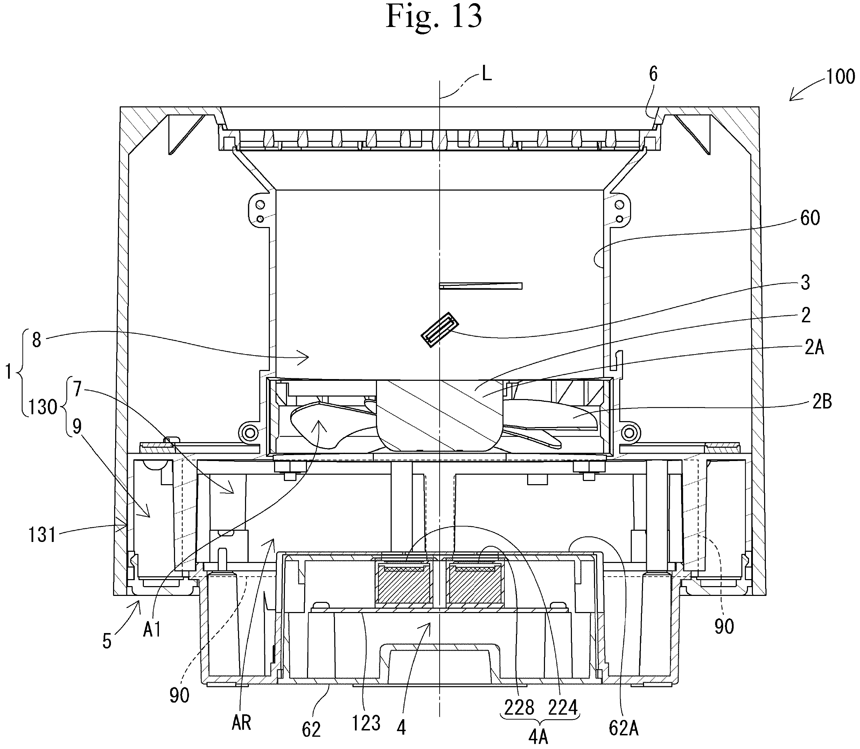

As shown in Fig. 13, the ozone sensor 4 is disposed in the flow path and upstream of the ozone generation unit 3. An upstream-side flow path 130 is formed by the guiding flow path 7 and the inlet-side flow path 9 of the flow path 1. The upstream-side flow path 130 forms a gas passing space AR on the upstream side of the ozone generation unit 3. The upstream-side flow path 130 allows gas to flow from one side toward the other side in a predetermined direction. The "predetermined direction" refers to the Z direction (up-down direction), the "one side" refers to the lower side, and "the other side" refers to the upper side. The ozone sensor 4 is disposed in the bottom portion 62. The bottom portion 62 has a case-like shape, and has a circuit substrate 123 disposed therein. The ozone sensor 4 is disposed on the circuit substrate 123. An upper wall portion 62A of the bottom portion 62 insulates the gas passing space AR from the lower side. The upper surface (surface on the other end side in the Z-axis direction) of the upper wall portion 62A is substantially orthogonal to the Z-axis direction (up-down direction). The upper wall portion 62A has a first opening 62B and a second opening 62C. The first opening 62B communicates with a first introduction inlet 231A of a first storage portion 231 described below. The second opening 62C communicates with a second introduction inlet 232A of a second storage portion 232 described below.

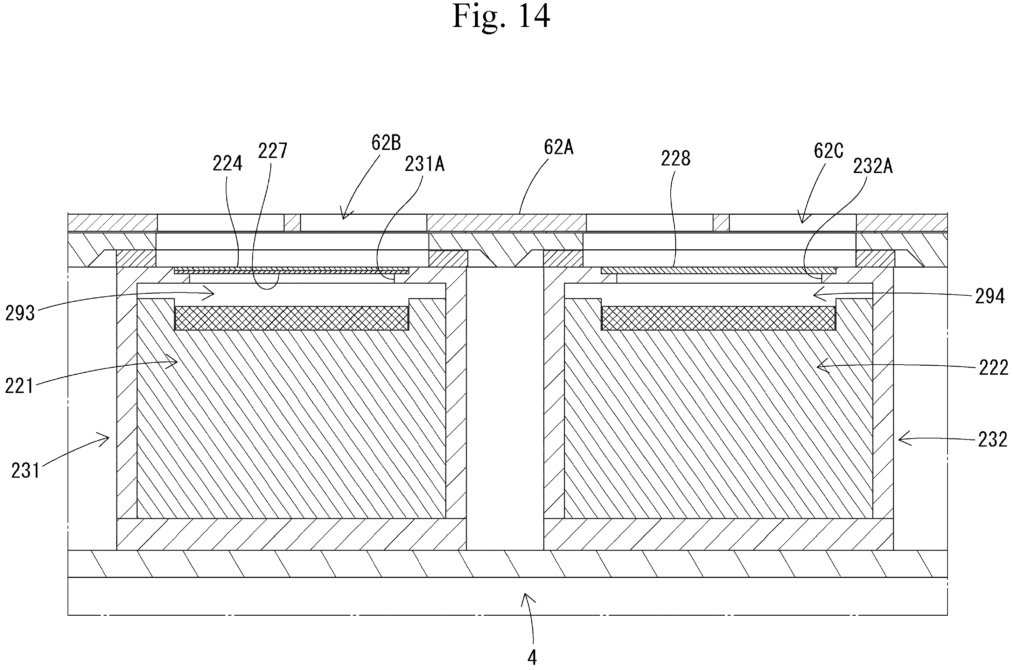

The ozone sensor 4 shown in Fig. 14 detects a concentration of ozone gas contained in a target gas. The ozone sensor 4 mainly includes a sensing portion 212 and a controller 214. The sensing portion 212 includes an element portion 220 and an output circuit portion 216. The controller 214 may be configured as the same controller as the controller 80 described above. The ozone sensor 4 detects a concentration of ozone gas that is contained in gas entering through the flow path 1.

The element portion 220 generates a first signal V1 (hereinafter, may also be referred to as voltage V1) as a signal for evaluating an electric current caused by difference in an amount of water vapor, and a second signal V2 (hereinafter, may also be referred to as voltage V2) as a signal corresponding to a concentration of ozone gas contained in a target gas. As shown in Fig. 14, the element portion 220 includes a first sensing element 221, a second sensing element 222, the first storage portion 231, the second storage portion 232, the first introduction inlet 231A, the second introduction inlet 232A, a moisture permeable film 224, a first waterproof filter 227, and a second waterproof filter 228.

The first storage portion 231 is a portion for storing the first sensing element 221 thereinside (in the internal space 293). The first storage portion 231 has the first introduction inlet 231A. The first introduction inlet 231A is an opening formed in the first storage portion 231. The first introduction inlet 231A is formed so as to penetrate, in the up-down direction, the upper wall portion forming a part of the first storage portion 231.

The moisture permeable film 224 is a filter disposed in the first introduction inlet 231A. The moisture permeable film 224 is a film body that has a function of taking moisture in a space in the flow path 1 thereinto (into the film) and guiding, to the internal space 293, moisture corresponding to the taken moisture, and a film body that substantially does not allow permeation of a tobe-detected gas. For example, the moisture permeable film 224 may be configured to take in moisture in a space in the flow path 1, cause the moisture to pass therethrough, and guide the moisture to the internal space 293. Alternatively, the moisture permeable film 224 may be configured to take in moisture in a space in the flow path 1, perform ion exchange thereinside, and guide moisture generated by the ion exchange to the internal space 293. For the moisture permeable film 224, polystyrene sulfonate, polyvinyl alcohol, vinyl alcohol copolymers, a fluorinebased ion exchange resin, a resin having a protic hydrophilic group in a repeating unit, a resin having an aprotic hydrophilic group in a repeating unit, or the like can be used. Examples of the fluororesin-based ion exchange membrane include Nafion (registered trademark), Flemion (registered trademark), and Aciplex (registered trademark). The moisture permeable film 224 may be used overlapping with a hydrophobic porous membrane. That is, any configuration may be used as long as an absolute humidity in the internal space 293 and an absolute humidity in a space in the flow path 1 can be made approximately equal to each other by presence of the moisture permeable film 224 disposed in the first introduction inlet 231A.

In the example in Fig. 14, the moisture permeable film 224 is formed by a water vapor permeable filter, and substantially prevents permeation of ozone gas from the flow path 1 to the internal space 293. In the moisture permeable film 224, a permeation amount of ozone gas is 1/50 or less of a permeation amount of water vapor in terms of volume. The moisture permeable film 224 is provided so as to close the first introduction inlet 231A. Therefore, gas entering the internal space 293 from the flow path 1 passes through the moisture permeable film 224 and enters the internal space 293.

The first waterproof filter 227 is a filter that allows gas such as water vapor and ozone to pass therethrough, and substantially prevents liquid from passing therethrough. In the first waterproof filter 227, a permeation amount of liquid is 1/50 or less of a permeation amount of gas in terms of volume. The first waterproof filter 227 is provided so as to close the first introduction inlet 231A. Therefore, gas entering the internal space 293 from the flow path 1 passes through the first waterproof filter 227 and enters the internal space 293.

The second storage portion 232 stores the second sensing element 222 thereinside (in the internal space 294). The second storage portion 232 has the second introduction inlet 232A.

The second waterproof filter 228 is a filter that allows gas such as water vapor and ozone to pass therethrough, and substantially prevents liquid from passing therethrough. In the second waterproof filter 228, a permeation amount of liquid is 1/50 or less of a permeation amount of gas in terms of volume. The second waterproof filter 228 is provided so as to close the second introduction inlet 232A. Therefore, gas entering the internal space 294 from the flow path 1 passes through the second waterproof filter 228 and enters the internal space 294.

Each of the first sensing element 221 and the second sensing element 222 is an element that electrochemically causes an electric current corresponding to a concentration of ozone (ozone gas) to flow therethrough. However, each of the first sensing element 221 and the second sensing element 222 is also an element that generates an electromotive force corresponding to difference between an amount of water vapor inside the element and an amount of water vapor outside the element. The first sensing element 221 and the second sensing element 222 have the same configuration. In each of the first sensing element 221 and the second sensing element 222, when ozone gas is present in a space therearound, a reduction reaction occurs in its own detection electrode (not shown) and an oxidation reaction occurs in its own counter electrode (not shown). Therefore, an electric current corresponding to an ozone gas concentration flows due to such reactions. However, in addition to an electric current corresponding to an ozone gas concentration, each of the first sensing element 221 and the second sensing element 222 also causes flow of an "electric current caused by difference in an amount of water vapor". Specifically, in the first sensing element 221 and the second sensing element 222, when there is a difference between an amount of water vapor in the gas flowing in from the flow path 1 and an amount of water vapor inside the element (space inside the element or electrolyte in the element), an electric current corresponding to the difference is generated.

For example, in the first sensing element 221, an electric current corresponding to an ozone gas concentration in the internal space 293 and an electric current caused by difference in an amount of water vapor between inside and outside the first sensing element 221 flow between a detection electrode and a counter electrode thereof. Specifically, the above-described reaction occurs in an electrolyte in the first sensing element 221 due to the ozone gas supplied to the first sensing element 221 via the internal space 293, and thereby an electric current corresponding to an ozone gas concentration flows, and, in addition to the electric current, an electric current caused by difference in an amount of water vapor between inside and outside the first sensing element 221 flows. The relationship between an ozone gas concentration in the internal space 293 and an "electric current corresponding to an ozone gas concentration" generated in the first sensing element 221 is specified by a predetermined arithmetic expression, and the higher an ozone gas concentration in the internal space 293 is, the higher the "electric current corresponding to an ozone gas concentration" generated in the first sensing element 221 is. However, flow of ozone gas into the internal space 293 is substantially prevented by the moisture permeable film 224. Therefore, in the first sensing element 221, an electric current corresponding to the ozone gas concentration does not flow, or even if the electric current flows, it is extremely low.

Similarly, in the second sensing element 222, an electric current corresponding to an ozone gas concentration in the internal space 294 and an electric current caused by difference in an amount of water vapor between inside and outside the second sensing element 222 flow between a detection electrode and a counter electrode thereof. Specifically, the above-described reaction occurs in an electrolyte in the second sensing element 222 due to the ozone gas supplied to the second sensing element 222 via the internal space 294, and thereby an electric current corresponding to an ozone gas concentration flows, and, in addition to the electric current, an electric current caused by difference in an amount of water vapor between inside and outside the second sensing element 222 flows. The relationship between an ozone gas concentration in the internal space 294 and an "electric current corresponding to an ozone gas concentration" generated in the second sensing element 222 is specified by a predetermined arithmetic expression, and the higher an ozone gas concentration in the internal space 294 is, the higher the "electric current corresponding to an ozone gas concentration" generated in the second sensing element 222 is. In a case where difference in an amount of water vapor between inside and outside the first sensing element 221 and difference in an amount of water vapor between inside and outside the second sensing element 222 are approximately equal to each other, an "electric current caused by difference in an amount of water vapor" generated in the second sensing element 222 and an "electric current caused by difference in an amount of water vapor" generated in the first sensing element 221 are approximately equal to each other.

The output circuit portion 216 includes a first output circuit 216A and a second output circuit 216B. The first output circuit 216A has resistances R11 and R12 and an operational amplifier OP1. Ra represents a resistance value of the resistance R11. Rb represents a resistance value of the resistance R12. The first output circuit 216A is a circuit that converts an electric current Ia (hereinafter, also referred to as output Ia) generated in the first sensing element 221 to a voltage Va, and outputs a voltage V1 obtained by amplifying the voltage Va at a predetermined amplification factor (Rb/Ra). In the first output circuit 216A, the electric current Ia generated in the first sensing element 221 flows through the resistance R11, and the voltage Va (Va=Ia×Ra) corresponding to the electric current Ia is generated between both ends of the resistance R11. A relationship among the voltage Va, the voltage V1 outputted by the first output circuit 216A to the controller 214, and the electric current Ia generated in the first sensing element 221 satisfies

$$V1=Va\times Rb/Ra=Ia\times Rb.$$

Similarly, the second output circuit 216B has resistances R21 and R22 and an operational amplifier OP2. Rc represents a resistance value of the resistance R21. Rd represents a resistance value of the resistance R22. The second output circuit 216B is a circuit that converts an electric current Ib (hereinafter, also referred to as output Ib) generated in the second sensing element 222 to a voltage Vb, and outputs a voltage V2 obtained by amplifying the voltage Vb at a predetermined amplification factor (Rd/Rc). In the second output circuit 216B, the electric current Ib generated in the second sensing element 222 flows through the resistance R21, and the voltage Vb (Vb=Ib×Rc) corresponding to the electric current Ib is generated between both ends of the resistance R21. A relationship among the voltage Vb, the voltage V2 outputted by the second output circuit 216B to the controller 214, and the electric current Ib generated in the second sensing element 222 satisfies V2=Vb×Rd/Rc=Ib×Rd.

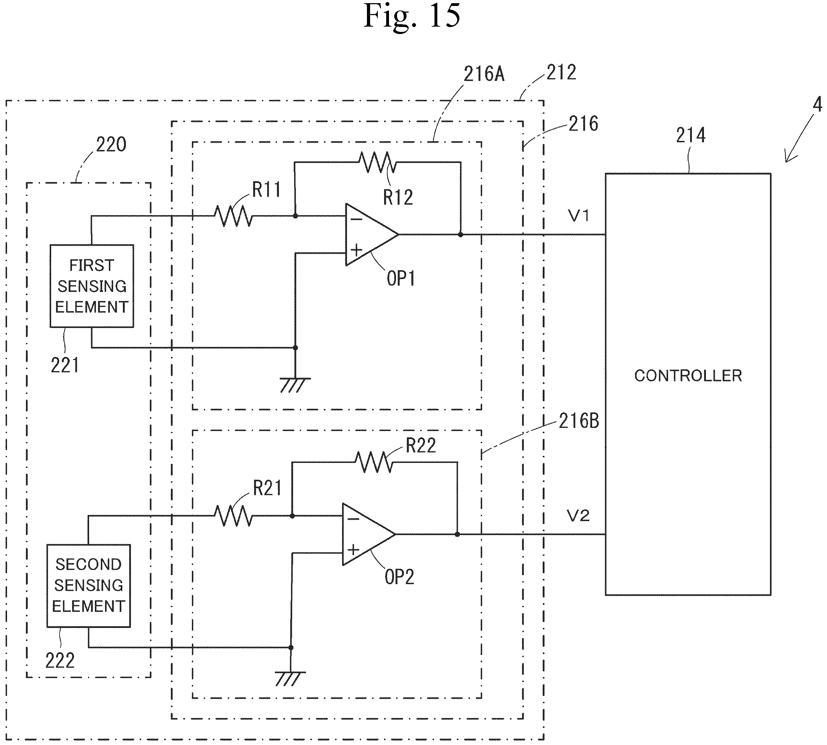

The controller 214 shown in Fig. 15 is configured as an information processing device having a function of performing an arithmetic operation, an information processing function, a control function, and the like. The controller 214 includes an AD converter, an MCU (micro controller unit), and the like. To the controller 214, the voltage V1 is inputted from the first output circuit 216A, and the voltage V2 is inputted from the second output circuit 216B. The controller 214 can convert the voltages V1 and V2 to digital signals, and can perform an arithmetic operation using the voltages V1 and V2.

In the ozone sensor 4, for example, Ra=Rc and Rb=Rd are satisfied, and an amplification factor at a first output circuit 16A and an amplification factor at a second output circuit 16B are equal to each other. The first sensing element 221 and the second sensing element 222 are the same. In the first sensing element 221, an electric current caused by difference in an amount of water vapor (electric current corresponding to difference in an amount of water vapor between inside and outside the first sensing element 221) flows. In the second sensing element 222, in addition to an electric current caused by difference in an amount of water vapor (electric current corresponding to difference in an amount of water vapor between inside and outside the second sensing element 222), and an electric current corresponding to an ozone concentration in a space in the flow path 1 is superimposed and flows. Therefore, the value of V2-V1 is a value obtained by cancelling influence of an "electric current caused by difference in an amount of water vapor" generated in the first sensing element 221 and the second sensing element 222, and is a voltage value corresponding to an ozone gas concentration in a space in the flow path 1.

In the sensing portion 212, a value of V2-V1 and an ozone gas concentration Y1 have such a correlation that the higher the ozone gas concentration Y1 in a space in the flow path 1 is, the greater the value of V2-V1 is. The correlation can be represented by, for example, a relational expression (linear expression) of Y1=β(V2-V1)+B. In the relational expression, the value of B is a predetermined fixed value (constant). The value of β may be a predetermined fixed value (constant), but may be a variable which is determined based on an absolute humidity. That is, the value of the gradient β in the relational expression (linear expression) may be corrected according to an absolute humidity. The controller 214 can perform an arithmetic operation for calculating an ozone gas concentration based on the above-described relational expression and the voltages V1 and V2. When the ozone gas concentration is calculated, the controller 214 may adopt the calculated concentration Y1 as it is, but it is desirable to apply a moving average assuming that the delay in response may occur. For example, in a case where the ozone gas concentration Y1 is repeatedly detected cyclically at predetermined time intervals, a simple moving average may be calculated so as to obtain an average of the most recent n pieces of data (the concentrations Yl), and the value may be adopted as an ozone gas concentration.

In this way, the ozone sensor 4 can cancel influence of water vapor based on the output Ia of the first sensing element 221 and the output Ib of the second sensing element 222 to obtain the ozone gas concentration Y1. Specifically, as described above, the value of V2-V1 is determined according to the expression of V1=Rb×Ia and the expression of V2=Rd×Ib=Rb×Ib. As described above, the value of V2-V1 is a value obtained by canceling influence of an "electric current caused by difference in an amount of water vapor" generated in the first sensing element 221 and the second sensing element 222, is a voltage value corresponding to an ozone gas concentration in a space in the flow path 1, and is a value represented by the expression of Rb(Ib-Ia). That is, the ozone sensor 4 can cancel influence of water vapor based on difference (Ib-Ia) between the output Ia and the output Ib to detect an ozone gas concentration.

1-5. Arrangement configuration of ozone sensor 4

As shown in Fig. 13, the inlet 5 is disposed closer to an outer circumferential portion 131 of the upstream-side flow path 130 than the ozone sensor 4. The outer circumferential portion 131 is formed by the second component 102 and the third component 103 of the flow path forming portion 60, and the intake portion 65. The outer circumferential portion 131 has an annular shape. The center axis of the outer circumferential portion 131 is coaxial with a rotation axis L of the fan 2. The outer circumferential portion 131 surrounds the upper end side of the bottom portion 62. The ozone sensor 4 is disposed on the center side (center axis side) of the outer circumferential portion 131.

As indicated by an arrow A1 shown in Fig. 13, airflow passing through the upstream-side flow path 130 passes through the inlet-side flow path 9 and the guiding flow path 7, and is thereafter bent upward, and passes between the rotor 2A and the first component 101 in the outlet-side flow path 8. The inlet 5 is disposed closer to the outer circumferential portion 131 than the ozone sensor 4. Therefore, airflow from the inlet 5 toward the other side (upper side) in a predetermined direction is unlikely to go toward the ozone sensor 4 located on the center side of the outer circumferential portion 131. That is, after passing through the guiding flow path 7, the airflow goes on a route to a direction away from the ozone sensor 4 (upward direction), without reaching the center side of the outer circumferential portion 131 (the center axis side of the fan 2). Therefore, influence of airflow in the ozone sensor 4 can be reduced.

As shown in Fig. 13, the filter 90 is disposed closer to the center of the upstream-side flow path 130 than the inlet 5. Therefore, gas entering through the inlet 5 flows through the filter 90 toward the outlet-side flow path 8. The ozone sensor 4 is disposed closer to the center of the upstream-side flow path 130 than the filter 90. Thus, gas passes through the filter 90, whereby airflow is weakened. Therefore, influence of airflow in the ozone sensor 4 can be further reduced.

The ozone sensor 4 overlaps the fan 2 when viewed in the rotation axis direction (the axial direction of the rotation axis L) of the fan 2. That is, the ozone sensor 4 is disposed below the fan 2. Since the direction from the inlet 5 toward the fan 2 is different from the rotation axis direction in which the ozone sensor 4 and the fan 2 overlap each other, airflow from the inlet 5 toward the fan 2 is unlikely to go toward the ozone sensor 4. Therefore, influence of airflow in the ozone sensor 4 can be further reduced.

The ozone sensor 4 is disposed in a range extending from the center of the rotor 2A over a distance of less than or equal to 1.2 times the diameter of the rotor 2A when viewed in the rotation axis direction of the fan 2. The ozone sensor 4 more preferably overlaps the rotor 2A when viewed in the rotation axis direction of the fan 2. In such a configuration, the ozone sensor 4 can be easily disposed while avoiding a position at which flow of gas is unlikely to occur (position overlapping the rotor 2A in the rotation axis direction). Therefore, influence of airflow in the ozone sensor 4 can be further reduced.

The ozone sensor 4 overlaps the outlet 6 when viewed in the rotation axis direction of the fan 2. The ozone sensor 4 is disposed such that a sensing surface 4A described below faces the outlet 6.

As shown in Fig. 13, the sensing surface 4A of the ozone sensor 4 is formed by the moisture permeable film 224 and the second waterproof filter 228. The sensing surface 4A of the ozone sensor 4 is disposed closer to the fan 2 than the inlet 5 in the rotation axis direction. Thus, the ozone sensor 4 can be easily stored inside the ozone generator 100 (on the downstream side), and the size of the ozone generator 100 can be easily reduced. More specifically, the sensing surface 4A of the ozone sensor 4 is disposed closer to the inlet 5 than the center of the length in the rotation axis direction of the fan 2 between the inlet 5 and the fan 2. That is, the height position of the sensing surface 4A of the ozone sensor 4 is lower than the center of the length between the height of the inlet 5 and the height of the fan 2 (for example, the height of the lower end of the fan 2). In such a configuration, the sensing surface 4A of the ozone sensor 4 can be easily kept away from airflow generated between the inlet 5 and the fan 2. Therefore, the ozone sensor 4 is much less likely to be influenced by airflow.

As shown in Fig. 13, the sensing surface 4A of the ozone sensor 4 is exposed to the flow path forming portion 60 (more specifically, gas passing space AR). That is, the sensing surface 4A of the ozone sensor 4 faces toward a route (the side where airflow is generated) of gas passing through the gas passing space AR. The sensing surface 4A of the ozone sensor 4 faces the other end side in the Z direction. The sensing surface 4A of the ozone sensor 4 is substantially parallel to the upper surface (surface on the other end side in the Z-axis direction) of the upper wall portion 62A. The sensing surface 4A of the ozone sensor 4 is substantially parallel to the lower surface (surface on the one end side in the Z direction) of the second component 102.

As shown in Fig. 13, the fan 2 is disposed in the outlet-side flow path 8 having a diameter less than a diameter of the outer circumferential portion 131. The outlet-side flow path 8 corresponds to an example of "small-diameter flow path" of the present disclosure. Thus, the fan 2 generates strong airflow in the outlet-side flow path 8 which is relatively narrow, whereas the ozone sensor 4 is disposed upstream of the fan 2. Therefore, ozone sensor 4 is less likely to be influenced by strong airflow.

As shown in Fig. 13, the plurality of the inlets 5 is arranged annularly (more specifically, in a round-ring shape) along the circumferential direction of the outer circumferential portion 131. Therefore, the ozone sensor 4 can be less likely to be influenced by any flow of gas entering through the plurality of the inlets 5, while an intake amount of gas is sufficiently ensured.

1-6. Effects of the first embodiment

In the first embodiment, the inlet 5 is disposed closer to the outer circumferential portion 131 of the upstream-side flow path 130 than the ozone sensor 4. Thus, in the upstream-side flow path 130 in which gas flows from one side to the other side in a predetermined direction, since the inlet 5 is disposed closer to the outer circumferential portion 131 than the ozone sensor 4, airflow from the inlet 5 toward the other side in the predetermined direction is unlikely to go toward the ozone sensor 4 located on the center side. Therefore, influence of airflow in the ozone sensor 4 can be reduced.

Furthermore, the ozone sensor 4 is disposed closer to the center of the upstream-side flow path 130 than the filter 90. Thus, gas passes through the filter 90, whereby airflow is weakened. Therefore, influence of airflow in the ozone sensor 4 can be further reduced.

Furthermore, the ozone sensor 4 overlaps the fan 2 when viewed in the rotation axis direction of the fan 2. Therefore, since the direction from the inlet 5 toward the fan 2 is different from the rotation axis direction in which the ozone sensor 4 and the fan 2 overlap each other, airflow from the inlet 5 toward the fan 2 is unlikely to go toward the ozone sensor 4. Therefore, influence of airflow in the ozone sensor 4 can be further reduced.

Furthermore, the ozone sensor 4 is disposed in a range extending from the center of the rotor 2A over a distance of less than or equal to 1.2 times the diameter of the rotor 2A when viewed in the rotation axis direction. Thus, the ozone sensor 4 can be easily disposed while avoiding a position at which flow of gas is unlikely to occur (position overlapping the rotor 2A of the fan 2 in the rotation axis direction). Therefore, influence of airflow in the ozone sensor 4 can be further reduced.

Furthermore, the sensing surface 4A of the ozone sensor 4 is disposed closer to the inlet 5 than the center of the length in the rotation axis direction of the fan 2 between the inlet 5 and the fan 2. Thus, the sensing surface 4A of the ozone sensor 4 can be easily kept away from airflow generated between the inlet 5 and the fan 2. Therefore, the ozone sensor 4 is much less likely to be influenced by airflow.

Furthermore, the sensing surface 4A of the ozone sensor 4 is disposed closer to the fan 2 than the inlet 5 in the rotation axis direction. Thus, the ozone sensor 4 can be easily stored inside the ozone generator 100 (on the downstream side), and the size of the ozone generator 100 can be easily reduced.

Furthermore, the fan 2 is disposed in the outlet-side flow path 8 having a diameter less than a diameter of the outer circumferential portion 131. Thus, the fan 2 generates strong airflow in the outlet-side flow path 8 which is relatively narrow, whereas the ozone sensor 4 is disposed upstream of the fan 2. Therefore, the ozone sensor 4 is less likely to be influenced by strong airflow.

Furthermore, the plurality of the inlets 5 is arranged annularly along the circumferential direction of the outer circumferential portion 131. Therefore, the ozone sensor 4 is less likely to be influenced by any flow of gas entering through the plurality of the inlets 5, while an intake amount of gas is sufficiently ensured.

Furthermore, the ozone sensor 4 is an electrochemical gas sensor. Therefore, measurement can be performed based on an electric current flowing according to an ozone gas concentration. Particularly, in an electrochemical gas sensor, when a sensing surface is exposed to airflow, the detection result is likely to be influenced by the airflow. Therefore, in a case where an electrochemical gas sensor is used, the effect of reducing influence of airflow in the ozone sensor 4 is more effectively exhibited by applying the configuration of the ozone generator 100 according to the present disclosure.

Furthermore, in the ozone sensor 4, the first sensing element 221 detects an ozone gas concentration in a state where ozone gas is substantially removed from a target gas, so that influence of an electric current caused by difference in an amount of water vapor can be evaluated. Meanwhile, the second sensing element 222 can detect a concentration of a target component containing water vapor and ozone gas. Thus, the ozone sensor 4 can utilize a detection result of the first sensing element 221 (result of evaluating influence of an electric current caused by difference in an amount of water vapor) for detecting a concentration of the ozone gas by the second sensing element 222, which is advantageous in correctly detecting a concentration of the ozone gas.

In the first embodiment, the holding portion 50 has a Young's modulus less than that of each of the first dielectric 11 and the second dielectric 31. Therefore, even when the first dielectric 11 or the second dielectric 31 vibrates, stress is less likely to be applied to a portion held by the holding portion 50. Accordingly, the first dielectric 11 and the second dielectric 31 are less likely to be broken.

Furthermore, the first dielectric 11 and the second dielectric 31 are each held at the one end on the same side. Therefore, an opening can be formed between the first dielectric 11 and the second dielectric 31 on the other end side in the longitudinal direction. Therefore, gas easily enters the discharge space DS formed between the first dielectric 11 and the second dielectric 31, and as a result, efficiency of generating ozone can be improved.

Furthermore, the holding portion 50 has the spacer 51 disposed between the first dielectric 11 and the second dielectric 31. Therefore, a distance between the first dielectric 11 and the second dielectric 31 can be easily set by the spacer 51.

Furthermore, the holding portion 50 has the extending portion 54 that extends from the spacer portion 53 and is disposed between the first protrusion 22 and the second protrusion 42. Therefore, the first terminal 12 and the second terminal 32 can be more reliably insulated from each other.

Furthermore, since the third connection portion 23 of the first terminal 12 extends so as to be bent from the end of the first protrusion 22, the first terminal 12 can be prevented from being broadened in the protruding direction of the first protrusion 22. Also, since the fourth connection portion 43 of the second terminal 32 extends so as to be bent from the end of the second protrusion 42, the second terminal 32 can be prevented from being broadened in the protruding direction of the second protrusion 42.

Furthermore, the holding portion 50 has the holder 52 for holding the first dielectric 11 and the second dielectric 31 holding the spacer 51 therebetween. Therefore, a distance between the first dielectric 11 and the second dielectric 31 can be maintained constant by the spacer 51 and the holder 52 of the holding portion 50.

Furthermore, the holder 52 has a ring shape that surrounds the outer periphery of the first dielectric 11 and the second dielectric 31 holding the spacer 51 therebetween. Therefore, assembling is facilitated by inserting the first dielectric 11 and the second dielectric 31 holding the spacer 51 therebetween into a hole of the holder 52.

Furthermore, the holder 52 has the first cut portion 58 obtained by cutting so as to expose the first terminal 12 and the second terminal 32. Therefore, the first terminal 12 and the second terminal 32 are easily embedded with resin through the first cut portion 58.

Furthermore, the holder 52 has the second cut portion 59 obtained by cutting so as to expose the discharge space DS. Therefore, while the outer periphery of the first dielectric 11 and the second dielectric 31 is surrounded by the holder 52, flow of gas into the discharge space DS can be allowed through the second cut portion 59. Thus, an inflow amount of gas into the discharge space DS can be prevented from being reduced due to the provision of the holder 52.

Furthermore, the first dielectric 11 and the second dielectric 31 are ceramic, and the holding portion 50 is made of resin. Therefore, while the first dielectric 11 and the second dielectric 31 are formed of ceramics, the first dielectric 11 and the second dielectric 31 can be prevented from being broken in a case where stress is applied to a portion held by the holding portion 50 under vibration.

Furthermore, the natural frequency Fn of each of the first electrode 10 and the second electrode 30 is higher than or equal to 200 Hz. Therefore, in a state where vibration is applied from the outside during transportation or the like, vibration due to resonance can be suppressed to be small. As a result, stress applied to the first dielectric 11 and the second dielectric 31 during vibration is reduced, and the first dielectric 11 and the second dielectric 31 are less likely to be broken.

Furthermore, the holding portion 50 has the double-sided tape 55 for adhering the first dielectric 11 and the second dielectric 31 to the spacer 51. Therefore, the first dielectric 11 and the second dielectric 31 are easily adhered to the spacer 51.

Furthermore, the holding portion 50 of the ozone generation unit 3 holds the first dielectric 11 and the second dielectric 31 only at one end side in the longitudinal direction, and is held outside the wall surface 1A of the flow path 1. The first dielectric 11 and the second dielectric 31 of the ozone generation unit 3 are disposed so as to protrude to the inside of the wall surface 1A. Therefore, as compared with a configuration in which both ends are held or a configuration in which ends on alternate sides are each held, the structure for fixing the ozone generation unit 3 and the wiring can be concentrated, so that the structure of the ozone generator 100 can be simplified.

Hereinafter, the effect of the filter 90 will be described.

The flow path 1 has the outlet 6, the inlet 5 disposed outward of the outer circumference of the outlet 6, the guiding flow path 7 for guiding gas taken in through the inlet 5 inwardly of the inner circumference of the inlet 5, and the outlet-side flow path 8 for guiding gas guided by the guiding flow path 7 to the outlet 6. The filter 90 is disposed in the guiding flow path 7. Thus, since the filter 90 is disposed in the guiding flow path 7 for guiding gas taken in through the inlet 5 inwardly of the inner circumference of the inlet 5, the filter 90 is invisible or cannot be easily seen from the inlet 5. Therefore, the design can be prevented from being degraded due to the filter 90 being visible from the outside. In the description herein, "outward of the outer circumference of the outlet 6" means "outward of the outer circumference of the outlet 6 when viewed from the direction (the Z direction) in which the outlet 6 is opened", and it also can be said as "radially outward of the outlet 6 having a round shape (radially outward of the outlet 6 having a round shape when viewed from the direction in which the outlet 6 is opened)".

Furthermore, the inlet 5 is disposed along an annular shape outward of the outer circumference of the outlet 6. The guiding flow path 7 is disposed along an annular shape, and guides gas taken in through the inlet 5 inwardly of the inner circumference of the inlet 5. The filter 90 is disposed along an annular shape in the guiding flow path 7. Therefore, gas is taken into the flow path 1 through the entire circumference of the ozone generator 100, and a foreign object in the taken gas can be efficiently removed by the filter 90.

Furthermore, the filter 90 is disposed along the intake direction of the inlet 5. Therefore, the filter 90 is less likely to be visible from the inlet 5.

Furthermore, the ozone generator 100 includes the filter frame 91 to which the filter 90 is mounted, the mounting portion 110 to which the filter frame 91 is mounted, the intake portion 65 having the inlet 5, and the attachment portion 115 to which the intake portion 65 is detachably attached. The intake portion 65 prevents the filter frame 91 from being removed from the mounting portion 110 in a state where the intake portion 65 is attached to the attachment portion 115, and allows the filter frame 91 to be removed from the mounting portion 110 in a state where the intake portion 65 is detached from the attachment portion 115. Thus, since the filter frame 91 can be mounted and dismounted merely by detaching the intake portion 65, an operation of mounting and dismounting the filter frame 91 is facilitated.

Furthermore, the mounting portion 110 has the mounting groove 111 into which the filter frame 91 fits, and the filter frame 91 is mounted to the mounting portion 110 by fitting into the mounting groove 111. Therefore, the filter frame 91 is easily mounted to and dismounted from the mounting portion 110.

Furthermore, the filter frame 91 has the frame body 92 having an annular shape, and the tab 93 protruding outwardly from the outer circumferential surface of the frame body 92. Even in a case where the inner circumferential surface of the frame body 92 is adjacent to the bottom portion 62, removal is facilitated by pinching the tab 93 protruding from the outer circumferential surface of the frame body 92.

Furthermore, the filter 90 is segmented into a plurality of the segmented filters 90A in the circumferential direction. Therefore, the filter 90 is easily mounted.

Furthermore, the opening width WK1 of the mounting hole 94 in the axial direction of the inlet 5 disposed along an annular shape is greater than the opening width WK2 of the inlet 5 in the direction orthogonal to the axial direction. Thus, the collection area of the filter 90 can be made greater than the opening area of the inlet 5. Therefore, a function of the filter 90 for removing foreign objects can be prevented from being degraded while suppressing pressure loss due to the filter 90.

<Other embodiments>

The present invention is not limited to the embodiment described above with reference to the drawings, and, for example, the following embodiments are also included in the technical scope of the present invention. Various characteristics of the embodiment described above and embodiments described below may be combined in any way as long as there is no contradiction.

Although, in the above-described embodiment, the inlet 5 is disposed in the intake portion 65 forming the outer circumferential portion 131, the inlet 105 may be disposed in the third component 103. That is, the inlet 5 may be structured to be opened in the lateral direction orthogonal to the up-down direction instead of being opened downwardin the outer circumferential portion 131. In this case, the sensing surface 4A of the ozone sensor 4 is preferably disposed closer to the inlet 5 than the center of the length in the rotation axis direction of the fan 2 between the lower edge of the inlet 5 and the fan 2.

Although, in the above-described embodiment, the Z direction corresponds to the up-down direction, the Z direction may not necessarily correspond to the up-down direction. For example, the Z direction may be a direction diagonal to the up-down direction.

Although, in the above-described embodiment, the holding portion holds the first dielectric and the second dielectric at each one end thereof, the holding portion may hold the first dielectric and the second dielectric at both ends thereof.

Although, in the above-described embodiment, the holding portion holds the first dielectric and the second dielectric at each one end thereof on the same side, the holding portion may hold the first dielectric and the second dielectric, for example, at each one end on an alternate opposite side, instead of holding at each one end on the same side.

Although, in the above-described embodiment, the Z direction corresponds to the up-down direction, the Z direction may not necessarily correspond to the up-down direction. For example, the Z direction may be a direction diagonal to the up-down direction.

Although, in the above-described embodiment, the holding portion holds the first dielectric and the second dielectric at each one end thereof, the holding portion may hold the first dielectric and the second dielectric at both ends thereof.

Although, in the above-described embodiment, the holding portion holds the first dielectric and the second dielectric at each one end thereof on the same side, the holding portion may hold the first dielectric and the second dielectric, for example, at each one end on an alternate opposite side, instead of holding at each one end on the same side.

(Appendix)

The present invention can include the following aspects [1] to [19].

[1] An ozone generator including:

    a flow path through which gas flows from an inlet to an outlet;
    an ozone generation unit disposed in the flow path; and
    an ozone sensor disposed in the flow path and upstream of the ozone generation unit, in which
    the flow path has an upstream-side flow path that forms a gas passing space located upstream of the ozone generation unit and through which the gas flows from one side to another side in a predetermined direction, and
    the inlet is disposed closer to an outer circumferential portion of the upstream-side flow path than the ozone sensor.

[2] The ozone generator according to [1], in which

    a filter is disposed closer to a center of the upstream-side flow path than the inlet, and
    the ozone sensor is disposed closer to the center of the upstream-side flow path than the filter.

[3] The ozone generator according to [1] or [2] in which

    a fan for sending gas toward the outlet is disposed downstream of the inlet and the ozone sensor, and
    the ozone sensor overlaps the fan when viewed in a rotation axis direction of the fan.

[4] The ozone generator according to [3], in which

the fan has a rotor, and a blade portion protruding from the rotor in a radial direction, and
the ozone sensor is disposed in a range extending from a center of the rotor over a distance of less than or equal to 1.2 times a diameter of the rotor, when viewed in the rotation axis direction.

[5] The ozone generator according to any one of [1] to [4] in which

a fan for sending gas toward the outlet is disposed downstream of the inlet and the ozone sensor, and
a sensing surface of the ozone sensor is disposed closer to the inlet than a center of a length in the rotation axis direction of the fan between the inlet and the fan.

[6] The ozone generator according to any one of [1] to [5], in which

a fan for sending gas toward the outlet is disposed downstream of the inlet and the ozone sensor, and
the sensing surface of the ozone sensor is disposed closer to the fan than the inlet in the rotation axis direction of the fan.

[7] The ozone generator according to any one of [1] to [6], in which

a fan for sending gas toward the outlet is disposed downstream of the inlet and the ozone sensor,
the flow path has a small-diameter flow path having a diameter less than a diameter of the outer circumferential portion, and
the fan is disposed in the small-diameter flow path.

[8] The ozone generator according to any one of [1] to [7], in which a plurality of the inlets is arranged annularly along a circumferential direction of the outer circumferential portion.
[9] The ozone generator according to any one of [1] to [8], in which the ozone sensor is an electrochemical gas sensor.
[10] The ozone generator according to [9], in which

the ozone sensor is
a gas sensor for detecting a concentration of ozone gas contained in a target gas, and includes
a first sensing element for electrochemically detecting a concentration of gas,
a second sensing element for electrochemically detecting a concentration of gas,
a first storage portion having, in its own structure, an internal space for storing the first sensing element,
a first introduction inlet provided between the internal space and the flow path outside the first storage portion, and
a moisture permeable film disposed at the first introduction inlet and configured to substantially prevent permeation of the ozone gas from the flow path to the internal space, and
the second sensing element is disposed in the flow path into which water vapor and the ozone gas contained in the target gas flow.

[11] The ozone generator according to [10], in which the moisture permeable film is a water vapor permeable filter that allows permeation of water vapor from the flow path to the internal space, and substantially prevents permeation of the ozone gas from the flow path to the internal space.
[12] An ozone generator including

an ozone generation unit for generating ozone,
a flow path in which the ozone generation unit is disposed, and
a filter disposed in the flow path, in which
the flow path has an outlet, an inlet disposed outward of an outer circumference of the outlet, a guiding flow path for guiding gas taken in through the inlet inwardly of an inner circumference of the inlet, and an outlet-side flow path for guiding the gas guided by the guiding flow path to the outlet, and
the filter is disposed in the guiding flow path.

In the ozone generator according to [12], since the filter is disposed in the guiding flow path for guiding gas taken in through the inlet inwardly of the inner circumference of the inlet, the filter is invisible or cannot be easily seen from the inlet. Therefore, the design can be prevented from being degraded due to the filter being visible from the outside.
In the apparatus as disclosed in Patent Document 2, since the filter disposed inside is visible through the opening, the design may be degraded. One of the objects of the ozone generator according to [12] is to provide a technique capable of preventing the design from being degraded due to the filter being visible from the outside, and the design can be prevented from being degraded due to the filter being visible from the outside.

[13] The ozone generator according to [12], in which

the inlet is disposed along an annular shape outward of an outer circumference of the outlet,
the guiding flow path is disposed along an annular shape, and guides gas taken in through the inlet inwardly of an inner circumference of the inlet, and
the filter is disposed along an annular shape in the guiding flow path.

In the configuration of [13], gas is taken into the flow path through the entire circumference of the ozone generator, and a foreign object in the taken gas can be efficiently removed by the filter.

[14] The ozone generator according to [12] or [13], in which the filter is disposed along an intake direction of the inlet.

In the configuration of [14], the filter is less likely to be visible from the inlet.

[15] The ozone generator according to any one of [12] to [14], including:

a filter frame to which the filter is mounted;
a mounting portion to which the filter frame is mounted;
an intake portion having the inlet; and
an attachment portion to which the intake portion is detachably attached, in which
the intake portion prevents the filter frame from being removed from the mounting portion in a state where the intake portion is attached to the attachment portion, and allows the filter frame to be removed from the mounting portion in a state where the intake portion is detached from the attachment portion.

In the configuration of [15], since the filter frame can be mounted and dismounted merely by detaching the intake portion, an operation of mounting and dismounting the filter frame is facilitated.

[16] The ozone generator according to [15], in which

the mounting portion has a mounting groove into which the filter frame fits, and
the filter frame is mounted to the mounting portion by fitting into the mounting groove.

In the configuration of [16], the filter frame is easily mounted to and dismounted from the mounting portion.

[17] The ozone generator according to any one of [12] to [16], including a filter frame to which a filter is mounted, in which
the filter frame has a frame body having an annular shape, and a tab protruding outwardly from an outer circumferential surface of the frame body.

In the configuration of [17], even in a case where the inner circumferential surface of the frame body is adjacent to a member disposed inside and is not easily pinched, removal is facilitated by pinching the tab protruding from the outer circumferential surface of the frame body.

[18] The ozone generator according to [13], in which the filter is segmented into a plurality of segmented filters in the circumferential direction.

In the configuration of [18], the filter is easily mounted.

[19] The ozone generator according to any one of [12] to [18], including a filter frame having a mounting hole in which the filter is mounted, in which

the inlet is disposed along an annular shape outward of the outer circumference of the outlet,
the guiding flow path is disposed along an annular shape, and guides gas taken in through the inlet inwardly of the inner circumference of the inlet,
the filter is disposed along an annular shape in the guiding flow path, and
an opening width of the mounting hole in an axial direction of the inlet disposed along an annular shape is greater than an opening width of the inlet in a direction orthogonal to the axial direction.

In the configuration of [19], the collection area of the filter can be made greater than the opening area of the inlet. Therefore, a function of the filter for removing foreign objects can be prevented from being degraded while suppressing pressure loss due to the filter.

It should be understood that the embodiments disclosed herein are in all aspects illustrative and not restrictive. The scope of the present invention is not limited to the embodiments disclosed herein, and is intended to include all modifications within the scope of the claims or within the scope equivalent to the scope of the claims.

DESCRIPTION OF REFERENCE NUMERALS

1: flow path
2: fan
2A: rotor
2B: blade portion
3: ozone generation unit
4: ozone sensor
5: inlet
6: outlet
7: guiding flow path
8: outlet-side flow path (small-diameter flow path)
60: flow path forming portion
65: intake portion
90: filter
90A: segmented filter
91: filter frame
92: filter body
93: tab
94: mounting hole
100: ozone generator
110: mounting portion
113: partition
114: storage groove
115: attachment portion
120: intake portion body
121: attaching portion
130: upstream-side flow path
131: outer circumferential portion
221: first sensing element
222: second sensing element
224: moisture permeable film
231: first storage portion
231A: first introduction inlet
AR: gas passing space
WK1: opening width of mounting hole
WK2: opening width of inlet

**Claims**

1. An ozone generator comprising:

   a flow path through which gas flows from an inlet to an outlet;
   an ozone generation unit disposed in the flow path; and
   an ozone sensor disposed in the flow path and upstream of the ozone generation unit, wherein
   the flow path has an upstream-side flow path that forms a gas passing space located upstream of the ozone
   generation unit and through which the gas flows from one side to another side in a predetermined direction, and
   the inlet is disposed closer to an outer circumferential portion of the upstream-side flow path than the ozone
   sensor.

2. The ozone generator according to claim 1, wherein

   a filter is disposed closer to a center of the upstream-side flow path than the inlet, and

the ozone sensor is disposed closer to the center side of the upstream-side flow path than the filter.

3.  The ozone generator according to claim 1 or claim 2, wherein

    a fan for sending gas toward the outlet is disposed downstream of the inlet and the ozone sensor, and
    the ozone sensor overlaps the fan when viewed in a rotation axis direction of the fan.

4.  The ozone generator according to claim 3, wherein

    the fan has a rotor, and a blade portion protruding from the rotor in a radial direction, and
    the ozone sensor is disposed in a range extending from a center of the rotor over a distance of less than or equal to 1.2 times a diameter of the rotor, when viewed in the rotation axis direction.

5.  The ozone generator according to any one of claim 1 to claim 4, wherein

    a fan for sending gas toward the outlet is disposed downstream of the inlet and the ozone sensor, and
    a sensing surface of the ozone sensor is disposed closer to the inlet than a center of a length in the rotation axis direction of the fan between the inlet and the fan.

6.  The ozone generator according to any one of claim 1 to claim 5, wherein

    a fan for sending gas toward the outlet is disposed downstream of the inlet and the ozone sensor, and
    the sensing surface of the ozone sensor is disposed closer to the fan than the inlet in the rotation axis direction of the fan.

7.  The ozone generator according to any one of claim 1 to claim 6, wherein

    a fan for sending gas toward the outlet is disposed downstream of the inlet and the ozone sensor,
    the flow path has a small-diameter flow path having a diameter less than a diameter of the outer circumferential portion, and
    the fan is disposed in the small-diameter flow path.

8.  The ozone generator according to any one of claim 1 to claim 7, wherein a plurality of the inlets is arranged annularly along a circumferential direction of the outer circumferential portion.

9.  The ozone generator according to any one of claim 1 to claim 8, wherein the ozone sensor is an electrochemical gas sensor.

10. The ozone generator according to claim 9, wherein

    the ozone sensor is
    a gas sensor for detecting a concentration of ozone gas contained in a target gas, and includes
    a first sensing element for electrochemically detecting a concentration of gas,
    a second sensing element for electrochemically detecting a concentration of gas,
    a first storage portion having, in its own structure, an internal space for storing the first sensing element,
    a first introduction inlet provided between the internal space and the flow path outside the first storage portion, and
    a moisture permeable film disposed at the first introduction inlet and configured to substantially prevent permeation of the ozone gas from the flow path to the internal space, and
    the second sensing element is disposed in the flow path into which water vapor and the ozone gas contained in the target gas flow.

11. The ozone generator according to claim 10, wherein the moisture permeable film is a water vapor permeable filter that allows permeation of water vapor from the flow path to the internal space, and substantially prevents permeation of the ozone gas from the flow path to the internal space.

12. The ozone generator according to claim 2, wherein

    the flow path has an outlet, an inlet disposed outward of an outer circumference of the outlet, a guiding flow path for guiding gas taken in through the inlet inwardly of an inner circumference of the inlet, and an outlet-side flow path for guiding the gas guided by the guiding flow path to the outlet, and
the filter is disposed in the guiding flow path.

13. The ozone generator according to claim 12, wherein

the inlet is disposed along an annular shape outward of an outer circumference of the outlet,
the guiding flow path is disposed along an annular shape, and guides gas taken in through the inlet inwardly of an inner circumference of the inlet, and
the filter is disposed along an annular shape in the guiding flow path.

14. The ozone generator according to claim 12 or claim 13, wherein the filter is disposed along an intake direction of the inlet.

15. The ozone generator according to any one of claim 12 to claim 14, comprising:

a filter frame to which the filter is mounted;
a mounting portion to which the filter frame is mounted;
an intake portion having the inlet; and
an attachment portion to which the intake portion is detachably attached, wherein
the intake portion prevents the filter frame from being removed from the mounting portion in a state where the intake portion is attached to the attachment portion, and allows the filter frame to be removed from the mounting portion in a state where the intake portion is detached from the attachment portion.

# Fig. 1

# Fig. 2

# Fig. 3

# Fig. 4

# Fig. 5

# Fig. 6

# Fig. 7

# Fig. 8

# Fig. 9

# Fig. 10

# Fig. 11

100

| | | |
|---|---|---|
| OPERATION UNIT — 81 | | |
| OZONE DETECTOR — 82 | CONTROLLER — 80 | AC POWER SUPPLY — 74 → OZONE GENERATION UNIT — 3 |
| | | FAN — 2 |
| | | DISPLAY UNIT — 83 |
| | | SOUND OUTPUT UNIT — 84 |

# Fig. 12

# Fig. 13

Fig. 14

# Fig. 15

# Fig. 16

# Fig. 17

# Fig. 18

## INTERNATIONAL SEARCH REPORT

| International application No. |
|---|
| **PCT/JP2022/013844** |

**A.    CLASSIFICATION OF SUBJECT MATTER**

*C01B 13/11*(2006.01)i; *A61L 9/015*(2006.01)i; *G01N 27/27*(2006.01)i; *G01N 27/416*(2006.01)i; *B01D 46/10*(2006.01)i
FI:    C01B13/11 A; C01B13/11 F; A61L9/015; B01D46/10 A; G01N27/416 311L; G01N27/27 B; C01B13/11 Z; C01B13/11 K

According to International Patent Classification (IPC) or to both national classification and IPC

**B.    FIELDS SEARCHED**

Minimum documentation searched (classification system followed by classification symbols)

C01B13/11; A61L9/015; G01N27/27; G01N27/416; B01D46/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| A | Microfilm of the specification and drawings annexed to the request of Japanese Utility Model Application No. 022874/1988 (Laid-open No. 128820/1989) (NIPPON SHOKUBAI KAGAKU KOGYO CO., LTD.) 01 September 1989 (1989-09-01), entire text, all drawings | 1-15 |
| A | JP 2003-153995 A (SHARP CORP) 27 May 2003 (2003-05-27) entire text, all drawings | 1-15 |
| A | JP 2008-036168 A (TOSHIBA CORP) 21 February 2008 (2008-02-21) entire text, all drawings | 1-15 |
| A | JP 2018-130208 A (NGK SPARK PLUG CO) 23 August 2018 (2018-08-23) entire text, all drawings | 1-15 |
| A | JP 2002-075588 A (SHARP CORP) 15 March 2002 (2002-03-15) entire text, all drawings | 1-15 |
| A | JP 2000-140688 A (MATSUSHITA ELECTRIC IND CO LTD) 23 May 2000 (2000-05-23) entire text, all drawings | 1-15 |

☑ Further documents are listed in the continuation of Box C.      ☑ See patent family annex.

| *   | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **19 May 2022** | **31 May 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| International application No. |
| --- |
| **PCT/JP2022/013844** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2015-027368 A (FUJITSU GENERAL LTD) 12 February 2015 (2015-02-12)<br>entire text, all drawings | 1-15 |
| A | JP 2019-017538 A (NGK SPARK PLUG CO) 07 February 2019 (2019-02-07)<br>entire text, all drawings | 1-15 |

Form PCT/ISA/210 (second sheet) (January 2015)

**INTERNATIONAL SEARCH REPORT**
Information on patent family members

International application No.

**PCT/JP2022/013844**

| Patent document cited in search report | | | Publication date (day/month/year) | Patent family member(s) | | | Publication date (day/month/year) |
|---|---|---|---|---|---|---|---|
| JP | 1-128820 | U1 | 01 September 1989 | (Family: none) | | | |
| JP | 2003-153995 | A | 27 May 2003 | (Family: none) | | | |
| JP | 2008-036168 | A | 21 February 2008 | (Family: none) | | | |
| JP | 2018-130208 | A | 23 August 2018 | (Family: none) | | | |
| JP | 2002-075588 | A | 15 March 2002 | US entire text, all drawings WO EP CA CN | 2003/0072675 2001/087364 1293216 2409000 1420791 | A1 A1 A1 A1 A | |
| JP | 2000-140688 | A | 23 May 2000 | (Family: none) | | | |
| JP | 2015-027368 | A | 12 February 2015 | (Family: none) | | | |
| JP | 2019-017538 | A | 07 February 2019 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0549831 A

- JP H07323081 A